# EUROPEAN PATENT APPLICATION

(11) **EP 4 223 758 A1**
(43) Date of publication of application: **09.08.2023**
(21) Application number: 21874515.6
(22) Date of filing: 29.09.2021
(51) Int. Cl.: C07D 487/04, A61K 31/4188, A61P 35/00

(54) **CRYSTAL FORM OF PYRROLO HETEROCYCLIC DERIVATIVE AND PREPARATION METHOD THEREFOR**

(30) Priority: 29.09.2020 CN 202011049402
(71) Applicant: Jiangsu Hengrui Pharmaceuticals Co., Ltd., Lianyungang, Jiangsu 222047 (CN); Shanghai Hengrui Pharmaceutical Co., Ltd, Shanghai 200245 (CN)
(72) Inventor: ZHOU, Xianqiang, Lianyungang, Jiangsu 222047 (CN); SHAO, Cheng, Shanghai 200245 (CN); YOU, Lingfeng, Shanghai 200245 (CN); DU, Zhenxing, Lianyungang, Jiangsu 222047 (CN); WANG, Jie, Lianyungang, Jiangsu 222047 (CN)
(74) Representative: dompatent von Kreisler Selting Werner - Partnerschaft von Patent- und Rechtsanwälten mbB
(86) International application number: PCT/CN2021/121641
(87) International publication number: WO 2022/068860

(57) **Abstract**

Provided are a crystal form of a pyrrolo heterocyclic derivative and a preparation method therefor. Specifically, the present invention relates to a crystal form of a pharmaceutically acceptable salt of a compound as represented by formula (I) and a preparation method therefor. The provided crystal form of the pharmaceutically acceptable salt of the compound as represented by formula (I) has a good stability and can be better used in clinical treatment.

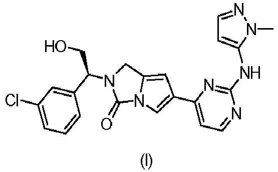

## Description

The present application claims priority to Chinese Patent Application No. 2020110494022 filed on Sep. 29, 2020, which is incorporated herein by reference in its entirety.

### TECHNICAL FIELD

The present disclosure relates to a novel crystal form of a pyrrolo heterocyclic derivative and a preparation method therefor and is in the field of pharmacy.

### BACKGROUND

The proliferation, differentiation, metabolism and apoptosis of normal cells are strictly regulated by the signal transduction pathways of cells in the body. Mitogen-activated protein kinases (MAPKs) play a crucial role in the signal transduction pathways. Extracellular signal regulated kinases (ERKs) are members of the MAPK family. Through the RAS-RAF-MEK-ERK step, exogenous stimulation signals are transmitted to ERKs. The activated ERKs are transferred into nuclei to regulate the activity of transcription factors and thus the biological functions of cells such as their proliferation, differentiation and apoptosis or, by phosphorylating the cytoskeleton in the cytoplasm, to participate in the regulation of cell morphology and the redistribution of the cytoskeleton.

Mutations in the RAS and RAF genes result in the sustained activation of the MAPK-ERK signaling pathway, promoting the malignant transformation and abnormal proliferation of cells and finally leading to the development of tumors (Roberts PJ, et al., Oncogene, 2007, 26(22), 3291-3310). The combination of a MEK inhibitor and a B-RAF inhibitor can further improve the inhibitory effect of the B-RAF inhibitor on tumor growth, and can significantly improve the progression-free survival and overall survival rate of patients with melanoma containing BRAFV600E and V600K mutations (Frederick DT et al., Clinical Cancer Research, 2013.19(5), 1225-1231). Although the combination of B-RAF/MEK inhibitors has the effect of inhibiting tumors, the effect is temporary: the majority of patients will develop resistance to the drugs within 2-18 months and the tumors will further progress. The mechanism for the development of resistance to B-RAF/MEK inhibitors is very complex and, in most cases, is directly related to the reactivation of the ERK signaling pathway (Smalley I et al., Cancer Discovery, 2018, 8(2), 140-142). Therefore, the development of new ERK inhibitors would benefit not only patients containing mutations in the MAPK signaling pathway but also patients with resistance to B-RAF/MEK inhibitors.

While inhibiting tumor growth, B-RAF/MEK inhibitors have a regulatory effect on the immune microenvironment of tumors. B-RAF/MEK inhibitors can enhance the expression of tumor-specific antigens, improve the recognition and killing of tumors by antigen-specific T cells, and promote the migration and infiltration of immune cells. In animal models, the expression of PD-L1 in tumor tissues is enhanced after treatment with B-RAF/MEK inhibitors, and when used in combination with a checkpoint molecule antibody (e.g., a PD-1 antibody or CTLA4 antibody), B-RAF/MEK inhibitors show better inhibitory effects on tumor growth than them alone (Boni A et al., Cancer Research, 2010, 70(13), 5213-5219). Research shows that ERK inhibitors are similar to B-RAF/MEK inhibitors in that when used in combination with a checkpoint antibody, they have the effect of regulating the microenvironment of tumors and can promote the functioning of cytotoxic T cells, thereby achieving the effect of inhibiting tumor growth. A number of compounds have now been developed. Among them, BVD-523 developed by BioMed Valley Discoveries Inc. is undergoing phase II clinical trials and MK-8353 by Merck & Co., Inc., as well as Astex-029 by Astex, is undergoing phase I clinical trials. The related patents include WO1999061440A1, WO2001056557A2, WO2001056993A2, WO2001057022A2, WO2002022601A1, WO2012118850A1, WO2013018733A1, WO2014179154A2, WO2015103133A1, WO2016192063A1, WO2017180817A1, and WO2018049127A1.

### SUMMARY

The present disclosure provides a pharmaceutically acceptable salt of a compound of formula (I), and the pharmaceutically acceptable salt is selected from the group consisting of a fumarate, an oxalate, a succinate, a maleate, a hydrochloride, and a hydrobromide,

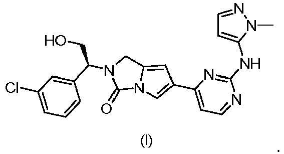

The present disclosure further provides a method for preparing a pharmaceutically acceptable salt of the compound of formula (I), which comprises the step of reacting the compound of formula (I) with a fumarate, an oxalate, a succinate, a maleate, a hydrochloride, or a hydrobromide.

In an alternative embodiment, the present disclosure provides a fumarate of the compound of formula (I), wherein the compound of formula (I) and fumaric acid are in a molar ratio of 1:1 or 2:1.

The present disclosure further provides a method for preparing a fumarate of the compound of formula (I), which comprises the step of reacting the compound of formula (I) with fumaric acid.

The present disclosure further provides crystal form I of a fumarate of the compound of formula (I), wherein the fumarate of the compound of formula (I) comprises the compound of formula (I) and fumaric acid in a molar ratio of 1:1 and, in an X-ray powder diffraction graph, has characteristic peaks at 2θ angles of diffraction of 10.671, 22.735, 28.681, and 29.302.

The present disclosure further provides crystal form I of a fumarate of the compound of formula (I), wherein the fumarate of the compound of formula (I) comprises the compound of formula (I) and fumaric acid in a molar ratio of 1:1 and, in an X-ray powder diffraction graph, has characteristic peaks at 2θ angles of diffraction of 10.671, 16.228, 19.832, 22.735, 23.526, 28.681, and 29.302.

The present disclosure provides in another aspect a method for preparing crystal form I of a fumarate of the compound of formula (I), wherein the fumarate of the compound of formula (I) comprises the compound of formula (I) and fumaric acid in a molar ratio of 1:1, the method comprising the following steps:
1) dissolving the compound of formula (I) and fumaric acid in a solvent selected from ethyl acetate; and
2) crystallization, wherein the compound of formula (I) and fumaric acid are in a molar ratio selected from 1:(1-1.5).

The present disclosure further provides crystal form II of a fumarate of the compound of formula (I), wherein the fumarate of the compound of formula (I) comprises the compound of formula (I) and fumaric acid in an amount-of-substance ratio of 1:1 and, in an X-ray powder diffraction graph, has characteristic peaks at 2θ angles of diffraction of 7.783, 9.151, 12.378, 14.808, 18.574, 25.755, and 26.802.

In an alternative embodiment, the present disclosure provides crystal form II of the fumarate of the compound of formula (I), wherein the fumarate of the compound of formula (I) comprises the compound of formula (I) and fumaric acid in an amount-of-substance ratio of 1:1 and, in an X-ray powder diffraction graph, has characteristic peaks at 2θ angles of diffraction of 7.783, 9.151, 10.292, 12.378, 14.808, 18.574, 19.632, 20.677, 22.305, 23.394, 25.755, 26.802, and 27.684.

In an alternative embodiment, the present disclosure provides crystal form II of the fumarate of the compound of formula (I), wherein the fumarate of the compound of formula (I) comprises the compound of formula (I) and fumaric acid in an amount-of-substance ratio of 1:1 and, in an X-ray powder diffraction graph, has characteristic peaks at 2θ angles of diffraction of 7.783, 9.151, 10.292, 12.378, 14.808, 16.386, 18.574, 19.632, 20.677, 22.305, 23.394, 24.053, 24.495, 25.755, 26.802, 27.684, 28.877, 29.681, and 31.851.

The present disclosure provides in another aspect a method for preparing crystal form II of a fumarate of the compound of formula (I), wherein the fumarate of the compound of formula (I) comprises the compound of formula (I) and fumaric acid in a molar ratio of 1:1, the method comprising the following steps:
1) dissolving the compound of formula (I) and fumaric acid in an ethanol or acetonitrile solution; and
2) crystallization, wherein the compound of formula (I) and fumaric acid are in a molar ratio selected from 1:(1-1.5).

In an alternative embodiment, the present disclosure provides crystal form III of a fumarate of the compound of formula (I), wherein the fumarate of the compound of formula (I) comprises the compound of formula (I) and fumaric acid in a molar ratio of 2:1 and, in an X-ray powder diffraction graph, has characteristic peaks at 2θ angles of diffraction of 4.877, 10.601, 13.827, 14.867, 16.272, 18.874, 23.921, and 26.367.

In an alternative embodiment, the present disclosure provides crystal form III of the fumarate of the compound of formula (I), wherein the fumarate of the compound of formula (I) comprises the compound of formula (I) and fumaric acid in a molar ratio of 2:1 and, in an X-ray powder diffraction graph, has characteristic peaks at 2θ angles of diffraction of 4.877, 9.664, 10.601, 11.745, 13.827, 14.867, 16.272, 17.885, 18.874, 20.695, 21.320, 22.204, 23.921, and 26.367.

In an alternative embodiment, the present disclosure provides crystal form IV of a fumarate of the compound of formula (I), wherein the fumarate of the compound of formula (I) comprises the compound of formula (I) and fumaric acid in a molar ratio of 2:1 and, in an X-ray powder diffraction graph, has characteristic peaks at 2θ angles of diffraction of 4.854, 10.613, 13.692, 23.523, 24.252, and 25.925.

In an alternative embodiment, the present disclosure provides crystal form IV of the fumarate of the compound of formula (I), wherein the fumarate of the compound of formula (I) comprises the compound of formula (I) and fumaric acid in a molar ratio of 2:1 and, in an X-ray powder diffraction graph, has characteristic peaks at 2θ angles of diffraction of 4.854, 9.785, 10.613, 13.692, 14.794, 18.424, 19.107, 20.409, 23.523, 24.252, and 25.925.

In an alternative embodiment, the present disclosure provides crystal form IV of the fumarate of the compound of formula (I), wherein the fumarate of the compound of formula (I) comprises the compound of formula (I) and fumaric acid in a molar ratio of 2:1 and, in an X-ray powder diffraction graph, has characteristic peaks at 2θ angles of diffraction of 4.854, 8.650, 9.785, 10.613, 11.658, 12.449, 13.692, 14.794, 15.393, 16.257, 17.864, 18.424, 19.107, 20.409, 21.322, 22.510, 23.523, 24.252, 25.925, and 28.382.

A method for preparing crystal form IV of a fumarate of the compound of formula (I) is provided, wherein the fumarate of the compound of formula (I) comprises the compound of formula (I) and fumaric acid in a molar ratio of 2:1; crystal form III of a fumarate of the compound of formula (I) is placed in an environment with humidity at greater than 50%.

In an alternative embodiment, the present disclosure provides an oxalate of the compound of formula (I).

The present disclosure further provides crystal form a of an oxalate of the compound of formula (I), which has characteristic peaks at 2θ angles of diffraction of 14.235, 15.983, 17.882, and 26.923 in an X-ray powder diffraction graph.

In an alternative embodiment, the present disclosure provides a maleate of the compound of formula (I), wherein the compound of formula (I) and maleic acid are in a molar ratio of 1:1.

The present disclosure further provides crystal form a of a maleate of the compound of formula (I), wherein the maleate of the compound of formula (I) comprises the compound of formula (I) and maleic acid in a molar ratio of 1:1 and, in an X-ray powder diffraction graph, has characteristic peaks at 2θ angles of diffraction of 4.510, 18.170, 23.973, 24.864, 26.306, and 28.209.

In an alternative embodiment, the present disclosure provides crystal form a of the maleate of the compound of formula (I), wherein the maleate of the compound of formula (I) comprises the compound of formula (I) and maleic acid in a molar ratio of 1:1 and, in an X-ray powder diffraction graph, has characteristic peaks at 2θ angles of diffraction of 4.510, 18.170, 19.959, 21.842, 23.973, 24.864, 26.306, 28.209, and 31.995.

The present disclosure provides in another aspect a method for preparing crystal form a of a maleate of the compound of formula (I), wherein the maleate of the compound of formula (I) comprises the compound of formula (I) and maleic acid in a molar ratio of 1:1, the method comprising the following steps: dissolving the compound of formula (I) and maleic acid in an isopropanol solution; and 2) crystallization, wherein the compound of formula (I) and maleic acid are in a molar ratio selected from 1:(1-1.5).

The present disclosure further provides crystal form b of a maleate of the compound of formula (I), wherein the maleate of the compound of formula (I) comprises the compound of formula (I) and maleic acid in a molar ratio of 1:1 and, in an X-ray powder diffraction graph, has characteristic peaks at 2θ angles of diffraction of 4.744, 10.024, 11.931, 15.969, 19.171, 25.291, and 28.006.

In an alternative embodiment, the present disclosure provides crystal form b of the maleate of the compound of formula (I), wherein the maleate of the compound of formula (I) comprises the compound of formula (I) and maleic acid in a molar ratio of 1:1 and, in an X-ray powder diffraction graph, has characteristic peaks at 2θ angles of diffraction of 4.744, 10.024, 11.931, 14.420, 15.185, 15.969, 17.249, 19.171, 20.405, 24.110, 25.291, 28.006, 30.216, and 32.380.

The present disclosure provides in another aspect a method for preparing crystal form b of a maleate of the compound of formula (I), wherein the maleate of the compound of formula (I) comprises the compound of formula (I) and maleic acid in a molar ratio of 1:1, the method comprising the following steps: dissolving the compound of formula (I) and maleic acid in an ethyl acetate solution; and 2) crystallization, wherein the compound of formula (I) and maleic acid are in a molar ratio selected from 1:(1-1.5).

The present disclosure further provides crystal form c of a maleate of the compound of formula (I), wherein the maleate of the compound of formula (I) comprises the compound of formula (I) and maleic acid in a molar ratio of 1:1 and, in an X-ray powder diffraction graph, has characteristic peaks at 2θ angles of diffraction of 4.610, 9.840, 10.708, 11.414, 12.329, 18.485, 18.883, 19.328, and 26.160.

In an alternative embodiment, the present disclosure provides crystal form c of the maleate of the compound of formula (I), wherein the maleate of the compound of formula (I) comprises the compound of formula (I) and maleic acid in a molar ratio of 1:1 and, in an X-ray powder diffraction graph, has characteristic peaks at 2θ angles of diffraction of 4.610, 9.840, 10.708, 11.414, 12.329, 14.346, 15.262, 16.655, 17.298, 18.485, 18.883, 19.328, 23.928, 26.160, and 28.389.

In an alternative embodiment, the present disclosure provides crystal form c of the maleate of the compound of formula (I), wherein the maleate of the compound of formula (I) comprises the compound of formula (I) and maleic acid in a molar ratio of 1:1 and, in an X-ray powder diffraction graph, has characteristic peaks at 2θ angles of diffraction of 4.610, 9.840, 10.708, 11.414, 12.329, 12.395, 14.346, 15.262, 15.962, 16.655, 17.298, 18.485, 18.883, 19.328, 22.250, 23.928, 26.160, 28.389, and 31.162.

The present disclosure provides in another aspect a method for preparing crystal form c of a maleate of the compound of formula (I), wherein the maleate of the compound of formula (I) comprises the compound of formula (I) and maleic acid in a molar ratio of 1:1, the method comprising the following steps: dissolving the compound of formula (I) and maleic acid in an acetonitrile solution; and 2) crystallization, wherein the compound of formula (I) and maleic acid are in a molar ratio selected from (10-2):1.

The present disclosure further provides crystal form d of a maleate of the compound of formula (I), wherein the maleate of the compound of formula (I) comprises the compound of formula (I) and maleic acid in a molar ratio of 1:1 and, in an X-ray powder diffraction graph, has characteristic peaks at 2θ angles of diffraction of 6.679, 9.346, 13.476, 14.076, 15.388, and 19.183.

In an alternative embodiment, the present disclosure provides crystal form d of the maleate of the compound of formula (I), wherein the maleate of the compound of formula (I) comprises the compound of formula (I) and maleic acid in a molar ratio of 1:1 and, in an X-ray powder diffraction graph, has characteristic peaks at 2θ angles of diffraction of 6.679, 8.405, 9.346, 10.170, 13.476, 14.076, 15.388, 19.183, 21.228, and 26.247.

In an alternative embodiment, the present disclosure provides crystal form d of the maleate of the compound of formula (I), wherein the maleate of the compound of formula (I) comprises the compound of formula (I) and maleic acid in a molar ratio of 1:1 and, in an X-ray powder diffraction graph, has characteristic peaks at 2θ angles of diffraction of 6.679, 8.405, 9.346, 10.170, 10.876, 13.476, 14.076, 15.388, 16.993, 19.183, 20.286, 21.228, 22.286, 23.894, 24.639, 26.247, 27.070, and 28.403.

The present disclosure further provides crystal form e of a maleate of the compound of formula (I), wherein the maleate of the compound of formula (I) comprises the compound of formula (I) and maleic acid in a molar ratio of 1:1 and, in an X-ray powder diffraction graph, has characteristic peaks at 2θ angles of diffraction of 6.193, 10.009, 13.646, 17.582, 17.996, 18.758, 22.733, and 24.766.

In an alternative embodiment, the present disclosure provides crystal form e of the maleate of the compound of formula (I), wherein the maleate of the compound of formula (I) comprises the compound of formula (I) and maleic acid in a molar ratio of 1:1 and, in an X-ray powder diffraction graph, has characteristic peaks at 2θ angles of diffraction of 6.193, 10.009, 13.646, 16.375, 17.582, 17.996, 18.758, 19.306, 20.181, 22.733, and 24.766.

In an alternative embodiment, the present disclosure provides crystal form e of the maleate of the compound of formula (I), wherein the maleate of the compound of formula (I) comprises the compound of formula (I) and maleic acid in a molar ratio of 1:1 and, in an X-ray powder diffraction graph, has characteristic peaks at 2θ angles of diffraction of 6.193, 10.009, 11.591, 13.646, 16.375, 17.582, 17.996, 18.758, 19.306, 20.181, 22.733, 24.766, 25.332, 26.601, 27.303, 28.859, and 31.447.

In an alternative embodiment, the present disclosure provides a succinate of the compound of formula (I).

The present disclosure further provides crystal form α of a succinate of the compound of formula (I), which has characteristic peaks at 2θ angles of diffraction of 5.640, 10.720, 13.384, 15.208, 16.244, 19.992, 21.669, 23.839, and 24.826 in an X-ray powder diffraction graph.

In an alternative embodiment, the present disclosure provides crystal form α of the succinate of the compound of formula (I), which has characteristic peaks at 2θ angles of diffraction of 5.640, 10.720, 13.384, 15.208, 16.244, 17.280, 18.020, 18.907, 19.992, 21.669, 23.839, and 24.826 in an X-ray powder diffraction graph.

The present disclosure further provides crystal form β of a succinate of the compound of formula (I), which has characteristic peaks at 2θ angles of diffraction of 5.160, 17.951, 20.618, 25.557, and 26.545 in an X-ray powder diffraction graph.

In an alternative embodiment, the present disclosure provides a hydrobromide of the compound of formula (I), wherein the compound of formula (I) and hydrobromic acid are in a molar ratio of 1:1.

The present disclosure further provides crystal form α of the hydrobromide of the compound of formula (I) described above, wherein the hydrobromide of the compound of formula (I) comprises the compound of formula (I) and hydrobromic acid in a molar ratio of 1:1 and, in an X-ray powder diffraction graph, has characteristic peaks at 2θ angles of diffraction of 6.656, 9.552, 14.110, 19.095, 20.092, 25.220, and 27.119.

In an alternative embodiment, the present disclosure provides crystal form α of the hydrobromide of the compound of formula (I), wherein the hydrobromide of the compound of formula (I) comprises the compound of formula (I) and hydrobromic acid in a molar ratio of 1:1 and, in an X-ray powder diffraction graph, has characteristic peaks at 2θ angles of diffraction of 6.656, 9.552, 13.778, 14.110, 19.095, 19.522, 20.092, 21.422, 25.220, 25.837, and 27.119.

In an alternative embodiment, the present disclosure provides crystal form α of the hydrobromide of the compound of formula (I), wherein the hydrobromide of the compound of formula (I) comprises the compound of formula (I) and hydrobromic acid in a molar ratio of 1:1 and, in an X-ray powder diffraction graph, has characteristic peaks at 2θ angles of diffraction of 6.656, 9.552, 13.778, 14.110, 19.095, 19.522, 20.092, 21.422, 22.276, 22.988, 23.653, 25.220, 25.837, 26.217, 27.119, 31.107, 31.867, 32.769, and 34.810.

In an alternative embodiment, the present disclosure provides a hydrobromide of the compound of formula (I), wherein the compound of formula (I) and hydrobromic acid are in a molar ratio of 1:2.

The present disclosure provides a method for a hydrobromide of the compound of formula (I), which comprises the step of reacting the compound of formula (I) with a hydrobromide.

The present disclosure further provides crystal form β of the hydrobromide of the compound of formula (I) described above, wherein the hydrobromide of the compound of formula (I) comprises the compound of formula (I) and hydrobromic acid in a molar ratio of 1:2 and, in an X-ray powder diffraction graph, has characteristic peaks at 2θ angles of diffraction of 7.363, 12.914, 15.392, 16.865, 17.217, 19.704, 21.999, 22.515, 24.769, 27.056, and 28.236.

In an alternative embodiment, the present disclosure provides crystal form β of the hydrobromide of the compound of formula (I) described above, wherein the hydrobromide of the compound of formula (I) comprises the compound of formula (I) and hydrobromic acid in a molar ratio of 1:2 and, in an X-ray powder diffraction graph, has characteristic peaks at 2θ angles of diffraction of 7.363, 12.914, 14.351, 15.392, 16.865, 17.217, 19.704, 21.999, 22.515, 24.769, 27.056, 28.236, and 29.234.

In an alternative embodiment, the present disclosure provides crystal form β of the hydrobromide of the compound of formula (I), wherein the hydrobromide of the compound of formula (I) comprises the compound of formula (I) and hydrobromic acid in a molar ratio of 1:2 and, in an X-ray powder diffraction graph, has characteristic peaks at 2θ angles of diffraction of 7.363, 12.914, 14.351, 15.392, 16.865, 17.217, 19.452, 19.704, 21.999, 22.515, 23.275, 24.213, 24.769, 27.056, 28.236, 29.234, 29.764, 31.251, and 32.887.

In an alternative embodiment, the present disclosure provides crystal form γ of the hydrobromide of the compound of formula (I) described above, wherein the hydrobromide of the compound of formula (I) comprises the compound of formula (I) and hydrobromic acid in a molar ratio of 1:2 and, in an X-ray powder diffraction graph, has characteristic peaks at 2θ angles of diffraction of 5.463, 16.567, 21.227, 25.784, 26.643, 28.321, and 33.788.

In an alternative embodiment, the present disclosure provides crystal form γ of the hydrobromide of the compound of formula (I) described above, wherein the hydrobromide of the compound of formula (I) comprises the compound of formula (I) and hydrobromic acid in a molar ratio of 1:2 and, in an X-ray powder diffraction graph, has characteristic peaks at 2θ angles of diffraction of 5.463, 16.567, 19.733, 21.227, 22.332, 25.014, 25.784, 26.643, 28.321, 30.298, 32.170, 33.788, 36.211, and 37.939.

In an alternative embodiment, the present disclosure provides a hydrochloride of the compound of formula (I), wherein the compound of formula (I) and hydrochloric acid are in a molar ratio of 1:1.

The present disclosure further provides crystal form I of the hydrochloride of the compound of formula (I) described above, wherein the hydrochloride of the compound of formula (I) comprises the compound of formula (I) and hydrochloric acid in a molar ratio of 1:1 and, in an X-ray powder diffraction graph, has characteristic peaks at 2θ angles of diffraction of 5.187, 9.407, 9.848, 10.388, 17.354, 18.041, and 26.234.

In an alternative embodiment, the present disclosure provides crystal form I of the hydrochloride of the compound of formula (I), wherein the hydrochloride of the compound of formula (I) comprises the compound of formula (I) and hydrochloric acid in a molar ratio of 1:1 and, in an X-ray powder diffraction graph, has characteristic peaks at 2θ angles of diffraction of 5.187, 8.131, 9.407, 9.848, 10.388, 17.354, 18.041, 18.924, 23.830, 25.449, 26.234, 26.724, 27.362, 27.902, 29.275, and 31.581.

The present disclosure further provides crystal form II of the hydrochloride of the compound of formula (I) described above, wherein the hydrochloride of the compound of formula (I) comprises the compound of formula (I) and hydrochloric acid in a molar ratio of 1:1 and, in an X-ray powder diffraction graph, has characteristic peaks at 2θ angles of diffraction of 13.728, 17.948, 22.678, 25.525, 27.356, and 29.543.

In an alternative embodiment, the present disclosure provides crystal form II of the hydrochloride of the compound of formula (I), wherein the hydrochloride of the compound of formula (I) comprises the compound of formula (I) and hydrochloric acid in a molar ratio of 1:1 and, in an X-ray powder diffraction graph, has characteristic peaks at 2θ angles of diffraction of 13.728, 17.948, 20.593, 22.678, 25.525, 27.356, and 29.543.

The present disclosure further provides crystal form III of the hydrochloride of the compound of formula (I) described above, wherein the hydrochloride of the compound of formula (I) comprises the compound of formula (I) and hydrochloric acid in a molar ratio of 1:1 and, in an X-ray powder diffraction graph, has characteristic peaks at 2θ angles of diffraction of 5.854, 8.983, 11.902, 13.181, 18.159, 20.071, 21.371, 24.180, 25.066, 26.469, 27.086, 27.729, and 28.615.

In an alternative embodiment, the present disclosure provides crystal form III of the hydrochloride of the compound of formula (I), wherein the hydrochloride of the compound of formula (I) comprises the compound of formula (I) and hydrochloric acid in a molar ratio of 1:1 and, in an X-ray powder diffraction graph, has characteristic peaks at 2θ angles of diffraction of 5.854, 8.983, 11.902, 13.181, 16.876, 18.159, 20.071, 21.371, 22.799, 24.180, 25.066, 26.469, 27.086, 27.729, 28.615, 29.827, 30.904, 31.935, 33.571, 34.905, and 37.265.

The present disclosure further provides crystal form IV of the hydrochloride of the compound of formula (I) described above, wherein the hydrochloride of the compound of formula (I) comprises the compound of formula (I) and hydrochloric acid in a molar ratio of 1:1 and, in an X-ray powder diffraction graph, has characteristic peaks at 2θ angles of diffraction of 5.693, 12.474, 14.474, 17.840, 22.231, 23.938, 26.866, and 29.207.

In an alternative embodiment, the present disclosure provides crystal form IV of the hydrochloride of the compound of formula (I), wherein the hydrochloride of the compound of formula (I) comprises the compound of formula (I) and hydrochloric acid in a molar ratio of 1:1 and, in an X-ray powder diffraction graph, has characteristic peaks at 2θ angles of diffraction of 5.693, 12.474, 14.474, 17.352, 17.840, 19.596, 22.231, 23.109, 23.938, 24.573, 26.866, 27.841, 29.207, 33.452, and 34.915.

The present disclosure further provides crystal form V of the hydrochloride of the compound of formula (I) described above, wherein the hydrochloride of the compound of formula (I) comprises the compound of formula (I) and hydrochloric acid in a molar ratio of 1:1 and, in an X-ray powder diffraction graph, has characteristic peaks at 2θ angles of diffraction of 7.476, 14.346, 15.181, 17.185, 21.606, 22.770, 24.279, 24.952, and 28.336.

In an alternative embodiment, the present disclosure provides crystal form V of the hydrochloride of the compound of formula (I), wherein the hydrochloride of the compound of formula (I) comprises the compound of formula (I) and hydrochloric acid in a molar ratio of 1:1 and, in an X-ray powder diffraction graph, has characteristic peaks at 2θ angles of diffraction of 7.476, 9.536, 13.447, 14.346, 15.181, 17.185, 18.422, 21.606, 22.770, 24.279, 24.952, 28.336, and 29.773.

In an alternative embodiment, the present disclosure provides crystal form V of the hydrochloride of the compound of formula (I), wherein the hydrochloride of the compound of formula (I) comprises the compound of formula (I) and hydrochloric acid in a molar ratio of 1:1 and, in an X-ray powder diffraction graph, has characteristic peaks at 2θ angles of diffraction of 7.476, 9.536, 12.349, 13.447, 14.346, 15.181, 15.639, 16.694, 17.185, 18.422, 18.859, 21.606, 22.770, 23.425, 24.279, 24.952, 26.990, 28.336, 28.772, 29.773, and 30.846.

In an alternative embodiment, for the crystal forms of the pharmaceutically acceptable salts of the compound of formula (I) described above, the 2θ angles have a margin of error of ±0.3.

In an alternative embodiment, for the crystal forms of the pharmaceutically acceptable salts of the compound of formula (I) described above, the 2θ angles have a margin of error of ±0.2.

In certain embodiments, the methods for preparing the crystal forms described herein also comprise a filtration step, a washing step, or a drying step.

The present disclosure also provides a pharmaceutical composition prepared from the pharmaceutically acceptable salt of the compound of formula (I) or the crystal form of the pharmaceutically acceptable salt described above.

The present disclosure also provides a pharmaceutical composition, which comprises the following components: i) the pharmaceutically acceptable salt of the compound of formula (I) or the crystal form of the pharmaceutically acceptable salt described above, and ii) optional pharmaceutically acceptable carriers, diluents or excipients.

The present disclosure also provides a method for preparing a pharmaceutical composition, which comprises the step of mixing the component i) and component ii) described above.

The present disclosure also provides use of the pharmaceutically acceptable salt of the compound of formula (I) described above, or the crystal form of the pharmaceutically acceptable salt of the compound of formula (I) described above, or the composition described above, or a composition prepared by the method described above, in preparing a medicament for treating or preventing cancer, inflammation, or other proliferative diseases, preferably cancer; the cancer is selected from the group consisting of melanoma, liver cancer, kidney cancer, lung cancer, nasopharyngeal carcinoma, colorectal cancer, colon cancer, rectal cancer, pancreatic cancer, cervical cancer, ovarian cancer, breast cancer, bladder cancer, prostate cancer, leukemia, head and neck squamous cell carcinoma, cervical cancer, thyroid cancer, lymphoma, sarcoma, neuroblastoma, brain tumor, myeloma, astrocytoma, and glioma.

As used herein, "2θ or 2θ angle" refers to the angle of diffraction, and *θ* refers to the Bragg angle in ° or degrees; the 2*θ* of each characteristic peak has a margin of error of ±0.20, and the error may be -0.20, -0.19, -0.18, -0.17, -0.16, -0.15, -0.14, -0.13, -0.12, -0.11, -0.10, -0.09, -0.08, -0.07, -0.06, -0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, or 0.20.

As used herein, "crystallization" includes, but is not limited to, stirring crystallization, trituration crystallization, and evaporative crystallization.

The drying described herein is generally performed at a temperature of 25-100 °C, preferably 40-70 °C, or may be performed under atmospheric pressure or reduced pressure.

In the present application, the step of reacting the compound of formula (I) with a fumarate, an oxalate, a succinate, a maleate, a hydrochloride, or a hydrobromide is optionally carried out in a suitable solvent, for example, an ester solvent, specifically ethyl acetate; for example, an alcohol solvent, specifically methanol, ethanol, or isopropanol; for example, a nitrile solvent, specifically acetonitrile or propionitrile; for example, a ketone solvent, specifically acetone; for example, an ether solvent, specifically tetrahydrofuran, dioxane, diethyl ether, or isopropyl ether; for example, an alkane solvent, specifically n-hexane, n-heptane, or water, or a mixed solvent of the solvents described above.

### BRIEF DESCRIPTION OF THE DRAWINGS

FIG. 1 shows an XRPD graph of the amorphous form of the compound of formula (I).
FIG. 2 shows an XRPD graph of crystal form I of the fumarate of the compound of formula (I).
FIG. 3 shows an XRPD graph of crystal form II of the fumarate of the compound of formula (I).
FIG. 4 shows a DSC graph of crystal form II of the fumarate of the compound of formula (I).
FIG. 5 shows a TGA graph of crystal form II of the fumarate of the compound of formula (I).
FIG. 6 shows a DVS graph of crystal form II of the fumarate of the compound of formula (I).
FIG. 7 shows an XRPD graph of crystal form II of the fumarate of the compound of formula (I) before and after DVS.
FIG. 8 shows an XRPD graph of crystal form III of the fumarate of the compound of formula (I).
FIG. 9 shows an XRPD graph of crystal form IV of the fumarate of the compound of formula (I).
FIG. 10 shows an XRPD graph of crystal form a of the oxalate of the compound of formula (I).
FIG. 11 shows an XRPD graph of crystal form a of the maleate of the compound of formula (I).
FIG. 12 shows an XRPD graph of crystal form b of the maleate of the compound of formula (I).
FIG. 13 shows an XRPD graph of crystal form c of the maleate of the compound of formula (I).
FIG. 14 shows an XRPD graph of the amorphous form of the maleate of the compound of formula (I).
FIG. 15 shows an XRPD graph of crystal form d of the maleate of the compound of formula (I).
FIG. 16 shows an XRPD graph of crystal form e of the maleate of the compound of formula (I).
FIG. 17 shows an XRPD graph of crystal form α of the succinate of the compound of formula (I).
FIG. 18 shows an XRPD graph of crystal form β of the succinate of the compound of formula (I).
FIG. 19 shows an XRPD graph of crystal form α of the hydrobromide of the compound of formula (I).
FIG. 20 shows an XRPD graph of crystal form β of the hydrobromide of the compound of formula (I).
FIG. 21 shows an XRPD graph of crystal form γ of the hydrobromide of the compound of formula (I).
FIG. 22 shows an XRPD graph of crystal form I of the hydrochloride of the compound of formula (I).
FIG. 23 shows an XRPD graph of crystal form II of the hydrochloride of the compound of formula (I).
FIG. 24 shows an XRPD graph of crystal form III of the hydrochloride of the compound of formula (I).
FIG. 25 shows an XRPD graph of crystal form IV of the hydrochloride of the compound of formula (I).
FIG. 26 shows an XRPD graph of crystal form V of the hydrochloride of the compound of formula (I).

### DETAILED DESCRIPTION

Hereinafter, the present invention will be explained in more detail with reference to the examples. The examples are only used to illustrate the technical solutions of the present invention, rather than limit the essence and scope of the present invention.

Test conditions for the instruments used in the experiment:
The structures of the compounds were determined by nuclear magnetic resonance (NMR) spectroscopy and/or mass spectrometry (MS). NMR shifts (δ) are given in 10⁻⁶ (ppm). NMR spectra were measured on Bruker AVANCE-400 nuclear magnetic resonance instrument or Bruker AVANCE NEO 500M, with deuterated dimethyl sulfoxide (DMSO-d6), deuterated chloroform (CDCl₃) and deuterated methanol (CD₃OD) as solvents and tetramethylsilane (TMS) as an internal standard.

Mass spectra (MS) were measured on Agilent 1200/1290 DAD-6110/6120 Quadrupole MS liquid chromatography-mass spectrometry system (manufacturer: Agilent; MS model: 6110/6120 Quadrupole MS),
waters ACQuity UPLC-QD/SQD (manufacturer: waters; MS model: waters ACQuity Qda Detector/waters SQ Detector), and
THERMO Ultimate 3000-Q Exactive (manufacturer: THERMO; MS model: THERMO Q Exactive).

High performance liquid chromatography (HPLC) analyses were performed on Agilent HPLC 1200DAD, Agilent HPLC 1200VWD and Thermo U3000 DAD high pressure liquid chromatographs.

Chiral HPLC analyses were performed on Agilent 1260 DAD high performance liquid chromatograph.

Preparative high performance liquid chromatography was performed on Waters 2545-2767, Waters 2767-SQ Detecor2, Shimadzu LC-20AP and Gilson GX-281 preparative chromatographs.

Preparative chiral chromatography was performed on Shimadzu LC-20AP preparative chromatograph.

Ion chromatography was performed on Thermo Scientific Dionex Intergrion, column model: DionexIonPacTM AS11-HC (4 µm, 4 × 250 cm). XRPD refers to X-ray powder diffraction detection: The measurement was conducted on BRUKER D8 Discovery X-ray diffractometer with a Cu anode (40 kV, 40 mA) and Cu-Kα radiation (λ = 1.5418 Å) used. Scan mode: *θ*/2*θ*, scan range (2q range): 5-50°.

DSC refers to differential scanning calorimetry: The measurement was conducted on METTLER TOLEDO DSC 3+ differential scanning calorimeter with a temperature ramp rate of 10 °C/min, specific temperature ranges shown in corresponding graphs (mostly 25-300 or 25-350 °C), and a nitrogen purge rate of 50 mL/min.

TGA refers to thermogravimetric analysis: The measurement was conducted on METTLER TOLEDO TGA 2 thermogravimetric analyzer with a temperature ramp rate of 10 °C/min, specific temperature ranges shown in corresponding graphs (mostly 25-300 °C), and a nitrogen purge rate of 50 mL/min.

DVS refers to dynamic vapor sorption: The measurement was conducted on SMS DVS Advantage; at 25 °C, the humidity was changed as follows: 50%-95%-0%-95%-50%, in steps of 10% (5% for the last step) (specific humidity ranges are shown in corresponding graphs, and those listed here were used in most cases); the criterion was dm/dt being no greater than 0.002%.

In the examples, a solution refers to an aqueous solution unless otherwise specified.

In the examples, the reaction temperature is room temperature, i.e., 20-30 °C, unless otherwise specified.

The monitoring of the reaction progress in the examples was conducted by thin layer chromatography (TLC). The developing solvent for reactions, the eluent system for column chromatography purification and the developing solvent system for thin-layer chromatography include: A: dichloromethane/methanol system. The volume ratio of the solvents was adjusted according to the polarity of the compound, or by adding a small amount of basic or acidic reagents such as triethylamine and acetic acid.

### Example 1

### (S)-2-(1-(3-Chlorophenyl)-2-hydroxyethyl)-6-(2-((1-methyl-1H-pyrazol-5-yl)amino)pyr imidin-4-yl)-1,2-dihydro-3H-pyrrolo[1,2-c]imidazol-3-one 1

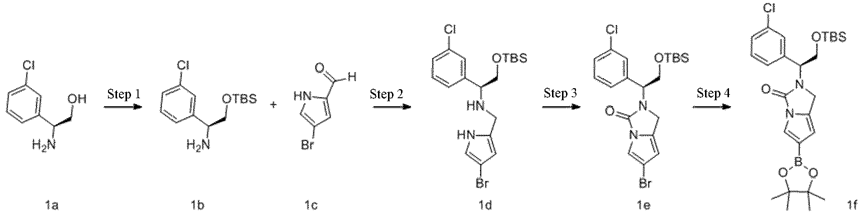

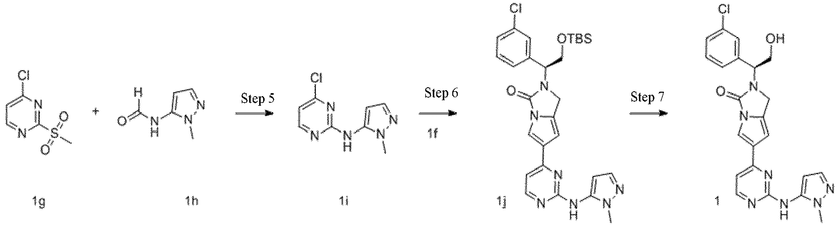

### Step 1

### (S)-2-((tert-Butyldimethylsilyl)oxy)-1-(3-chlorophenyl)ethylamine 1b

(*S*)-2-Amino-2-(3-chlorophenyl)ethanol **1a** (4 g, 23.3 mmol, Bide Pharmatech Ltd.) and imidazole (3.2 g, 46.6 mmol) were dissolved in 80 mL of dichloromethane, and tert-butyldimethylsilyl chloride (5.2 g, 35 mmol) was added under ice bath. The mixture was stirred for 14 h. Water was added, and extraction was performed with dichloromethane (80 mL × 3). The organic phases were combined, washed with saturated sodium chloride solution, dried over anhydrous sodium sulfate, and filtered. The filtrate was concentrated under reduced pressure and purified by column chromatography with eluent system C to give the title compound **1b** (6.5 g), yield: 97%. MS m/z (ESI): 286.1 [M+1]

### Step 2

### (S)-N-((4-Bromo-1H-pyrrol-2-yl)methyl)-2-((tert-butyldimethylsilyl)oxy)-1-(3-chloroph enyl)ethylamine 1d

4-Bromo-1*H*-pyrrole-2-carbaldehyde **1c** (2.37 g, 13.62 mmol, Bide Pharmatech Ltd.) and compound **1b** (3.9 g, 13.64 mmol) were stirred for 3 h. The mixture was diluted with 100 mL of methanol and cooled to 0 °C. Sodium borohydride (516 mg, 13.64 mmol) was added, and the mixture was stirred for 2 h. Water was added, and the reaction mixture was concentrated under reduced pressure. Water was added, and extraction was performed with ethyl acetate (40 mL × 3). The organic phases were combined, washed with saturated sodium chloride solution, dried over anhydrous sodium sulfate, and filtered. The filtrate was concentrated under reduced pressure and purified by column chromatography with eluent system C to give the title compound **1d** (4.8 g), yield: 79%.
MS m/z (ESI): 444.2 [M+1]

### Step 3

### (S)-6-Bromo-2-(2-((tert-butyldimethylsilyl)oxy)-1-(3-chlorophenyl)ethyl)-1H-pyrrolo[1 ,2-c]imidazol-3(2H)-one 1e

Compound **1d** (4.8 g, 10.81 mmol) was dissolved in 100 mL of tetrahydrofuran, and *N*,*N*'-carbonyldiimidazole (2.45 g, 15.11 mmol) was added under ice bath. The mixture was stirred for 0.5 h, and sodium hydride (60%, 621 mg, 16.22 µmol) was added. The mixture was stirred at room temperature for 14 h. Saturated ammonium chloride was added. The reaction mixture was concentrated under reduced pressure and purified by column chromatography with eluent system C to give the title compound **1e** (4.0 g), yield: 78%.
MS m/z (ESI): 469.1 [M+1]

### Step 4

### (S)-2-(2-((tert-Butyldimethylsilyl)oxy)-1-(3-chlorophenyl)ethyl)-6-(4,4,5,5-tetramethyl-1,3,2-dioxaborolan-2-yl)-1H-pyrrolo[1,2-c]imidazol-3(2H)-one 1f

Compound **1e** (4.0 g, 8.51 mmol) was dissolved in 50 mL of 1,4-dioxane under argon atmosphere, and 4,4,4',4',5,5,5',5'-octamethyl-2,2'-bis(1,3,2-dioxaborolane) (3.24 g, 12.76 mmol), potassium acetate (3.34 g, 34.04 mmol) and [1,1'-bis(diphenylphosphino)ferrocene]dichloropalladium (1.24 g, 1.70 mmol) were added in sequence. The mixture was stirred at 90 °C for 2 h, cooled, and filtered through celite. The filtrate was concentrated and purified by column chromatography with eluent system C to give the title compound **1f** (2.0 g), yield: 45%.
MS m/z (ESI): 517.2 [M+1]

### Step 5

### 4-Chloro-N-(1-methyl-1H-pyrazol-5-yl)pyrimidin-2-amine 1i

*N*-(1-methyl-1*H*-pyrazol-5-yl)formamide **1h** (324.82 mg, 2.60 mmol, prepared by the method disclosed in patent application "WO2017/80979") was dissolved in 15 mL of *N*,*N*-dimethylformamide, and sodium hydride (60%, 311.47 mg, 7.79 mmol) was added at 0 °C. The mixture was stirred for 0.5 h and, after 4-chloro-2-(methylsulfonyl)pyrimidine **1g**(500 mg, 2.60 mmol) was added, was reacted for another 2 h. Water (20 mL) was added, and extraction was performed with ethyl acetate (20 mL × 3). The organic phases were combined and concentrated under reduced pressure. The resulting residue was purified by thin-layer chromatography with developing solvent system C to give the title compound **1i** (270 mg), yield: 49.6%.
MS m/z (ESI): 210.3 [M+1]

### Step 6

### (S)-2-(2-((tert-Butyldimethylsilyl)oxy)-1-(3-chlorophenyl)ethyl)-6-(2-((1-methyl-1H-py razol-5-yl)amino)pyrimidin-4-yl)-1,2-dihydro-3H-pyrrolo[1,2-c]imidazol-3(2H)-one 1j

A mixture of compound **1f** (98.6 mg, 0.19 mmol), prepared 4-chloro-*N*-(1-methyl-1*H*-pyrazol-5-yl)pyrimidin-2-amine **1i**, [1,1'-bis(diphenylphosphino)ferrocene]dichloropalladium (28 mg, 0.02 mmol) and cesium carbonate (124 mg, 0.2 mmol) was suspended in 20 mL of 1,4-dioxane and 4 mL of water under argon atmosphere. The suspension was heated to 80 °C, stirred for 14 h, cooled, and filtered through celite. The filtrate was collected and extracted with ethyl acetate (20 mL × 3). The organic phases were combined, concentrated under reduced pressure, and purified by column chromatography with eluent system A to give the title compound **1j** (100 mg), yield: 92%.
MS m/z (ESI): 564.3 [M+1]

### Step 7

### (S)-2-(1-(3-Chlorophenyl)-2-hydroxyethyl)-6-(2-((1-methyl-1H-pyrazol-5-yl)amino)pyr imidin-4-yl)-1,2-dihydro-3H-pyrrolo[1,2-c]imidazol-3-one 1

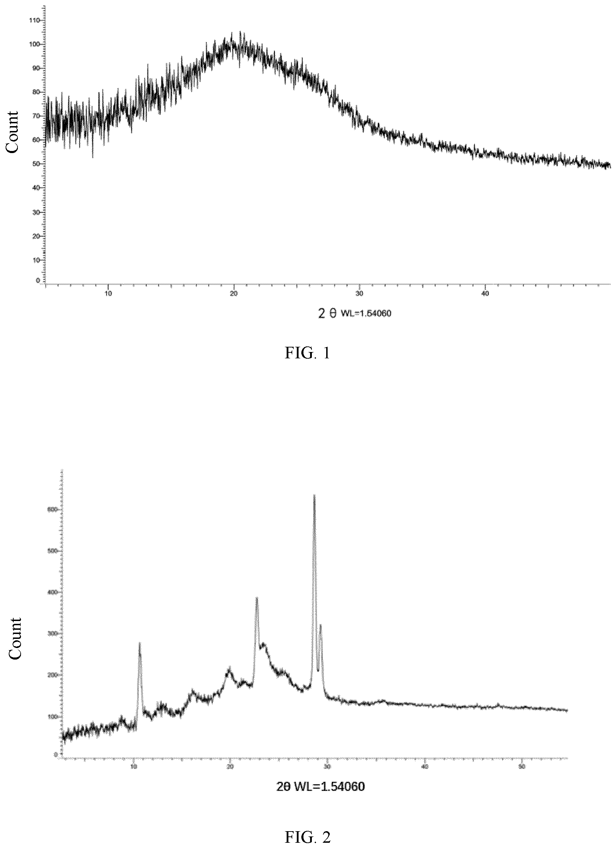

Compound **1j** (100 mg, 0.17 mmol) was dissolved in 20 mL of dichloromethane, and 1 mL of trifluoroacetic acid was added dropwise. After addition, the mixture was stirred for 4 h. The pH was adjusted to 7 with saturated sodium bicarbonate, and extraction was performed with dichloromethane (20 mL × 2). The organic phases were combined, concentrated under reduced pressure, and purified by column chromatography with eluent system A to give the title compound 1 (15 mg), yield: 18%. The product was identified by X-ray powder diffraction as amorphous. The XRPD graph is shown in FIG. 1.
MS m/z (ESI): 450.1 [M+1]
1H NMR (400 MHz, CDCl₃): δ 8.33 (d, 1H), 7.72 (s, 1H), 7.48 (d, 1H), 7.41-7.33 (m, 3H), 7.28-7.24 (m, 1H), 7.18 (s, 1H), 6.92 (d, 1H), 6.51 (s, 1H), 6.32 (d, 1H), 5.17 (dd, 1H), 4.46 (d, 1H), 4.32 (dd, 1H), 4.27-4.17 (m, 3H), 3.82 (s, 3H).

### Biological Evaluation

### Test Example 1: ERK1 Enzymatic Activity Test

### I. Objective

The objective of this experiment is to measure the ability of the compound to inhibit the enzymatic activity of ERK1 and evaluate the *in vitro* activity of the compound according to IC₅₀. This experiment used an ADP-Glo^{™} kinase assay kit. Under the action of enzyme, ADP was produced as the substrate was phosphorylated. The ADP-Glo reagent was added to remove unreacted ATP in the reaction system, and the ADP produced by the reaction was detected by a kinase detection reagent. In the presence of the compound, the inhibition rate of the compound was calculated by measuring the signal value.

### II. Method

Enzyme and substrate preparation: 0.75 ng/µL ERK1 (1879-KS-010, R&D) and 100 µM substrate (AS-61777, anaspec) were prepared in buffer, and the enzyme solution and substrate solution were mixed in a ratio by volume of 2:1 for later use. ATP was diluted with buffer to 300 µM. The compound was dissolved in DMSO (dimethyl sulfoxide, Shanghai Titan Scientific Co., Ltd.) to prepare a stock solution with an initial concentration of 20 mM, and the compound was prepared on Bravo. Finally, to each well of a 384-well plate were added in sequence 3 µL of the mixed solution of enzyme and substrate and 1 µL of the compound at different concentrations (an initial concentration of 50 µM, 4-fold dilutions). The plate was incubated at 30 °C for 10 min, and lastly, 1 µL of 300 µM ATP solution was added to each well. The plate was incubated at 30 °C for 2 h. Then 5 µL of ADP-Glo was added, and the plate was incubated at 30 °C for 40 min. Subsequently, 10 µL of kinase detection buffer was added, and the plate was incubated at 30 °C for 40 min. The 384-well plate was taken out and placed in a microplate reader (BMG labtech, PHERAstar FS) to measure chemiluminescence.

### III. Data analysis

The data were processed and analyzed using Microsoft Excel and Graphpad Prism 5. The IC₅₀ value of the compound was obtained. The result is shown in Table 1 below.

**Table 1. The IC₅₀ value of the compound of the present disclosure inhibiting the enzymatic activity of ERK1**

| Example No. | IC₅₀ (nM) |
|---|---|
| 1 | 5 |

Conclusion: the compound of the present disclosure has a significant inhibitory effect on the enzymatic activity of ERK1.

### Test Example 2: ERK2 Enzymatic Activity Test

### I. Objective

The objective of this experiment is to measure the ability of the compound to inhibit the enzymatic activity of ERK2 and evaluate the *in vitro* activity of the compound according to IC₅₀. This experiment used an ADP-Glo^{™} kinase assay kit. Under the action of enzyme, ADP was produced as the substrate was phosphorylated. The ADP-Glo reagent was added to remove unreacted ATP in the reaction system, and the ADP produced by the reaction was detected by a kinase detection reagent. In the presence of the compound, the inhibition rate of the compound was calculated by measuring the signal value.

### II. Method

Enzyme and substrate preparation: 0.75 ng/µL ERK2 (1230-KS-010, R&D) and 1500 µM substrate (custom polypeptide, GL Biochem) were prepared in buffer (40 mM Tris, 20 mM MgCl₂, 0.1 mg/mL BSA, 50 µM DTT), and the enzyme solution and substrate solution were mixed in a ratio by volume of 2:1 for later use. ATP was diluted with buffer to 500 µM. The compound was dissolved in DMSO (dimethyl sulfoxide, Shanghai Titan Scientific Co., Ltd.) to prepare a stock solution with an initial concentration of 20 mM, and the compound was prepared on Bravo. Finally, to each well of a 384-well plate were added in sequence 3 µL of the mixed solution of enzyme and substrate and 1 µL of the compound at different concentrations (an initial concentration of 50 µM, 4-fold dilutions). The plate was incubated at 30 °C for 10 min, and lastly, 1 µL of 500 µM ATP solution was added to each well. The plate was incubated at 30 °C for 2 h. Then 5 µL of ADP-Glo was added, and the plate was incubated at 30 °C for 40 min. Subsequently, 10 µL of kinase detection buffer was added, and the plate was incubated at 30 °C for 40 min. The 384-well plate was taken out and placed in a microplate reader (BMG labtech, PHERAstar FS) to measure chemiluminescence.

### III. Data analysis

The data were processed and analyzed using Microsoft Excel and Graphpad Prism 5. The IC₅₀ value of the compound was obtained. The result is shown in Table 2 below.

**Table 2. The IC₅₀ value of the compound of the present disclosure inhibiting the enzymatic activity of ERK2**

| Example No. | IC₅₀ (nM) |
|---|---|
| 1 | 7 |

Conclusion: the compound of the present disclosure has a significant inhibitory effect on the enzymatic activity of ERK2.

### Test Example 3: Test for Inhibition of In Vitro Proliferation of Colo205 Tumor Cells by the Compound

### I. Objective

The objective of this experiment is to measure the activity of the compound inhibiting the *in vitro* proliferation of Colo205 cells (CCL-222, ATCC). The cells were treated *in vitro* with different concentrations of the compound. After 3 days of culture, the proliferation of the cells was measured using CTG (CellTiter-Glo^{®} Luminescent Cell Viability Assay, Promega, catalog No. G7573) reagent, and the *in vitro* activity of the compound was evaluated according to the IC₅₀ value.

### II. Method

By way of example, a test for the inhibition of the *in vitro* proliferation of Colo205 cells is described below to illustrate the method of measuring the activity of the compound of the present disclosure inhibiting the *in vitro* proliferation of tumor cells. This method also applies to, but is not limited to, the measurement of the activity of inhibiting the *in vitro* proliferation of other tumor cells.

Colo205 was digested, centrifuged, and resuspended. The single cell suspension was well mixed, and the density of viable cells was adjusted with cell culture medium (RPMI1640 + 2% FBS) to 5.0 × 10⁴ cells/mL. The suspension was added to a 96-well cell culture plate at 95 µL/well. To the peripheral wells of the 96-well plate was added only 100 µL of medium. The culture plate was incubated in an incubator for 24 h (37 °C, 5% CO₂).

The compound was dissolved in DMSO (dimethyl sulfoxide, Shanghai Titan Scientific Co., Ltd.) to prepare a stock solution with an initial concentration of 20 mM. The small-molecule compound was 4-fold diluted from an initial concentration of 2 mM to 9 points. The tenth point was DMSO. To each well of another 96-well plate was added 90 µL of cell culture medium (RPMI1640 + 2% FBS), followed by 10 µL of test samples at different concentrations. The mixtures were well mixed, and 5 µL of test samples at different concentrations were then added to the cell culture plate. Two replicate wells were set for each sample. The culture plate was incubated in an incubator (37 °C, 5% CO₂) for 3 days. The 96-well cell culture plate was taken out, and 50 µL of CTG solution was added to each well. The plate was incubated at room temperature for 10 min and placed in a microplate reader (BMG labtech, PHERAstar FS) to measure chemiluminescence.

### III. Data analysis

The data were processed and analyzed using Microsoft Excel and Graphpad Prism 5. The result of this example is shown in Table 3 below.

**Table 3. The IC₅₀ value of the compound of the present disclosure inhibiting the in vitro proliferation of Colo205 tumor cells**

| Example No. | IC₅₀ (nM) |
|---|---|
| 1 | 62 |

### Pharmacokinetic Evaluation

### Test Example 4: Pharmacokinetic Study of the Compound of the Present Disclosure in Mice

### 1. Abstract

With mice as the test animals, the plasma concentration of the compounds of Examples 3, 10, 15 and 20 was determined by LC/MS/MS at different time points after intragastric administration. The pharmacokinetic behavior of the compound of the present disclosure in mice was studied and its pharmacokinetic profile was evaluated.

### 2. Methodology

### 2.1. Test drug

The compound of Example 1.

### 2.2. Test animals

Thirty-six C57 mice, female, evenly divided into 4 groups, purchased from Shanghai Jiesijie Laboratory Animal Co., Ltd., with animal production license number: SCXK (Shanghai) 2013-0006.

### 2.3. Drug preparation

A certain amount of the compound was measured out and dissolved in 5% by volume of DMSO and 5% Tween 80, and 90% normal saline was then added to prepare a 0.1 mg/mL colorless clear solution.

### 2.4. Administration

C57 mice were fasted overnight and then intragastrically administered the compound at a dose of 2 mg/kg and a volume of 0.2 mL/10 g.

### 3. Procedures

The mice were intragastrically administered the compound, and 0.1-mL blood samples were collected into heparin tubes before administration and 0.25 h, 0.5 h, 1.0 h, 2.0 h, 4.0 h, 6.0 h, 8.0 h, 11.0 h, and 24.0 h after administration. Plasma was separated by centrifuging the blood at 3500 rpm for 10 min, and was stored at -20 °C.

After different concentrations of the compound were injected into the mice, the plasma concentration of the compound was determined: 25 µL of mouse plasma at each time point post-dose was mixed with 50 µL of camptothecin (National Institutes for Drug Control) internal standard solution (100 ng/mL) and 200 µL of acetonitrile, and the mixture was vortexed for 5 min and centrifuged at 4000 rpm for 10 min; 4 µL of supernatant was taken for LC/MS/MS analysis.

### 4. Pharmacokinetic parameters

**Table 4. The pharmacokinetic parameters of the compound of the present disclosure**

| Example No. | Pharmacokinetic experiment in mice | | | | | |
|---|---|---|---|---|---|---|
| | Plasma concentration | Area under curve | Half-life | Residence time | Clearance | Apparent volume of distribution |
| | Cmax (ng /mL) | AUC (ng /mL*h) | T1/2 (h) | MRT (h) | CLz/F (ml/min/kg) | Vz/F (ml/kg) |
| Example 1 | 1023 | 3441 | 3.66 | 3.47 | 9.69 | 3067 |

Conclusion: the compound of the present disclosure demonstrated a good absorption profile; it has pharmacokinetic advantages.

### Example 2: Preparation of Fumarate in Crystal Form I

The amorphous compound of formula (I) (100 mg, 222.28 µmol) was added to 2 mL of ethyl acetate. The temperature was raised to 60 °C, and 29 mg of fumaric acid was added. The mixture was stirred for 30 min, gradually cooled to room temperature, and stirred at room temperature for 2 h. A solid gradually precipitated. The mixture was filtered under reduced pressure, and the filter cake was collected, washed with a small amount of ethyl acetate, and dried *in vacuo* at 40 °C to give a solid (101.1 mg, yield: 80.4%). The ¹H-NMR analysis of the product is shown below and indicates that the molar ratio of the compound of formula (I) to fumaric acid in the salt is 1:1.26.
¹H NMR (400 MHz, DMSO- *d₆*) δ 13.13 (br, 2H), 9.32 (s, 1H) 8.37 (d, 1H), 7.92 (s, 1H), 7.48 (d, 1H), 7.41-7.33 (m, 3H), 7.28-7.24 (m, 1H), 7.18 (s, 1H), 6.66 (d, 1H), 6.63 (s, 1H), 6.27 (s, 1H), 5.26 (s,1H), 5.17 (dd, 1H), 4.66 (d, 1H), 4.42 (dd, 1H), 3.94-4.06 (m, 2H), 3.82 (s, 3H).

The product was identified by X-ray powder diffraction as crystal form I. The XRPD graph is shown in FIG. 2, and the peak positions are shown in Table 5.

**Table 5. The peak positions of the fumarate in crystal form I**

| Peak number | 2θ(°) | d(Å) | I% |
|---|---|---|---|
| 1 | 10.671 | 8.28425 | 37.4 |
| 2 | 16.228 | 5.45762 | 8.2 |
| 3 | 19.832 | 4.47310 | 11.3 |
| 4 | 22.735 | 3.90809 | 42.9 |
| 5 | 23.526 | 3.77853 | 18.2 |
| 6 | 28.681 | 3.11002 | 100.0 |
| 7 | 29.302 | 3.04553 | 35.0 |

### Example 3: Preparation of Fumarate in Crystal Form II

The amorphous compound of formula (I) (100 mg, 222.28 µ µmol) was added to 2 mL of ethanol. The temperature was raised to 60 °C, and 29 mg of fumaric acid was added. The mixture was stirred for 30 min, cooled to room temperature, and stirred for another 2 h. A solid gradually precipitated. The mixture was filtered under reduced pressure, and the filter cake was collected, washed with a small amount of ethanol, and dried *in vacuo* at 40 °C to give a solid (80 mg, yield: 63.6%). The ¹H-NMR analysis of the product is shown below and indicates that the molar ratio of the compound of formula (I) to fumaric acid in the salt is 1:0.93.
¹H NMR (400 MHz, DMSO- *d₆*): δ 13.13 (br,2H), 9.32 (s,1H) 8.37 (d, 1H), 7.92 (s, 1H), 7.48 (d, 1H), 7.41-7.33 (m, 3H), 7.28-7.24 (m, 1H), 7.18 (s, 1H), 6.66 (d, 1H), 6.63 (s, 1H), 6.27 (s, 1H), 5.26 (s,1H), 5.17 (dd, 1H), 4.66 (d, 1H), 4.42 (dd, 1H), 3.94-4.06 (m, 2H), 3.82 (s, 3H).

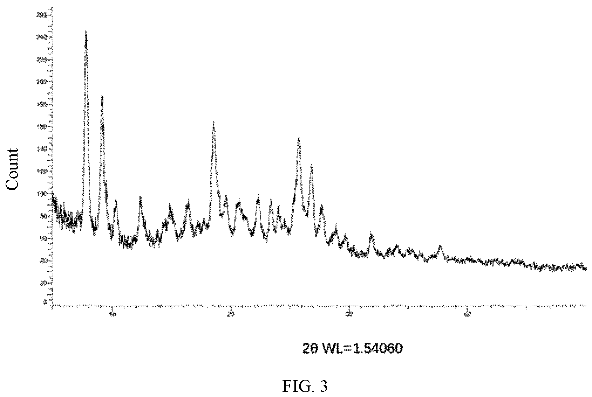

The product was identified by X-ray powder diffraction as crystal form II. The XRPD graph is shown in FIG. 3, and the peak positions are shown in Table 6.

**Table 6. The peak positions of the fumarate in crystal form II**

| Peak number | 2θ(°) | d(Å) | I(%) |
|---|---|---|---|
| 1 | 7.783 | 11.34974 | 100.00% |
| 2 | 9.151 | 9.65566 | 74.50% |
| 3 | 10.292 | 8.58777 | 18.80% |
| 4 | 12.378 | 7.14527 | 21.90% |
| 5 | 14.808 | 5.97759 | 13.40% |
| 6 | 16.386 | 5.40528 | 13.50% |
| 7 | 18.574 | 4.77319 | 53.80% |
| 8 | 19.632 | 4.51839 | 15.60% |
| 9 | 20.677 | 4.29231 | 12.20% |
| 10 | 22.305 | 3.98243 | 16.30% |
| 11 | 23.394 | 3.79950 | 16.90% |
| 12 | 24.053 | 3.69687 | 9.50% |
| 13 | 24.495 | 3.63113 | 2.80% |
| 14 | 25.755 | 3.45636 | 48.90% |
| 15 | 26.802 | 3.32358 | 35.00% |
| 16 | 27.684 | 3.21974 | 13.80% |
| 17 | 28.877 | 3.08937 | 5.20% |
| 18 | 29.681 | 3.00750 | 2.50% |
| 19 | 31.851 | 2.80732 | 4.80% |

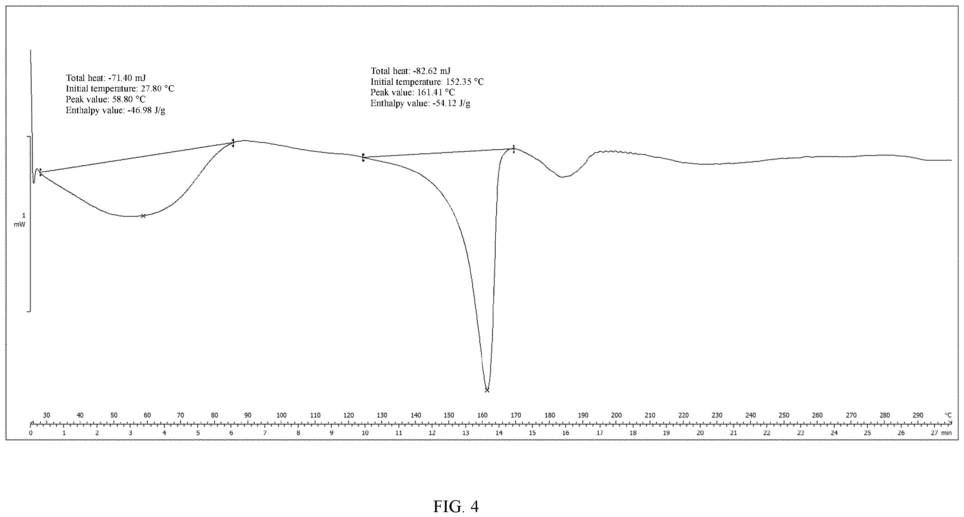

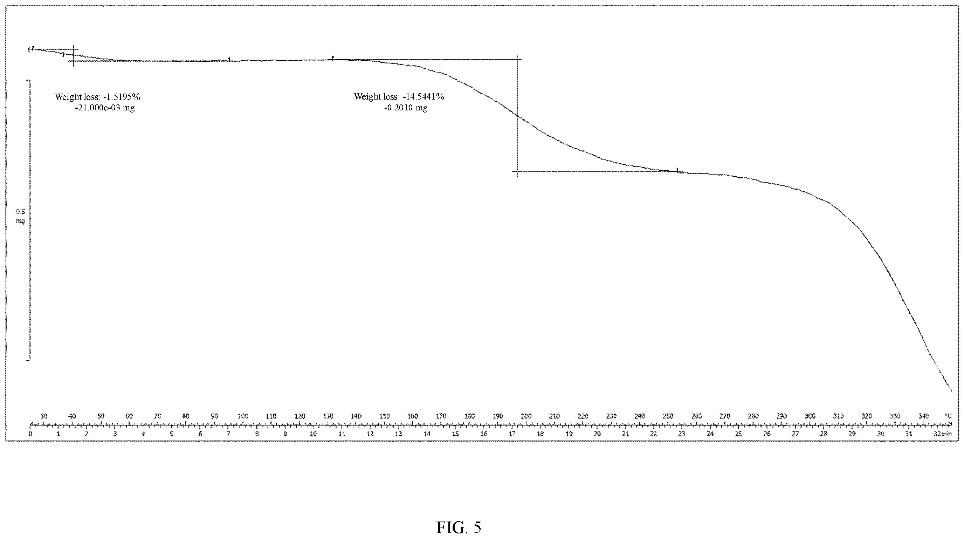

The DSC graph is shown in FIG. 4, where the endothermic peaks are at 58.80 °C and 161.41 °C. The TGA graph is shown in FIG. 5, where a weight loss of 1.52% is shown at 25-130 °C and a weight loss of 14.54% is shown at 130-280 °C.

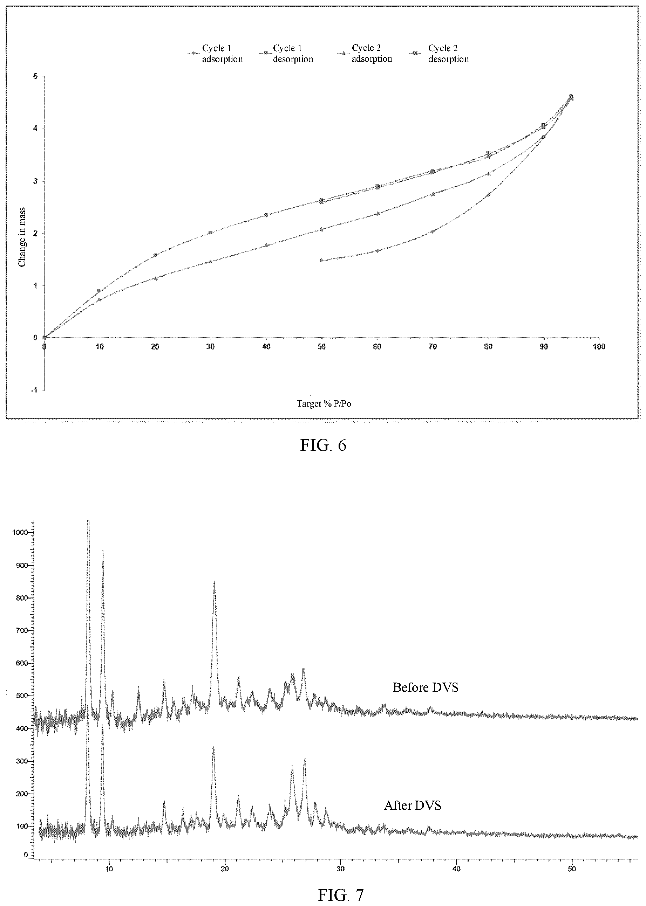

DVS analysis shows that the sample made a moisture-absorption weight gain of about 2.38% when stored under normal conditions (i.e., 25 °C, 60% RH), a moisture-absorption weight gain of about 2.75% when subjected to accelerated conditions (i.e., 70% RH), and a moisture-absorption weight gain of about 3.85% when subjected to extreme conditions (i.e., 90% RH). During the process of the RH changing from 0% to 95%, the desorption and adsorption of this sample did not coincide (see FIG. 6). After DVS analysis, the sample was re-examined for crystal form, and the result shows that the crystal form did not change (see FIG. 7).

### Example 4: Preparation of Fumarate in Crystal Form III

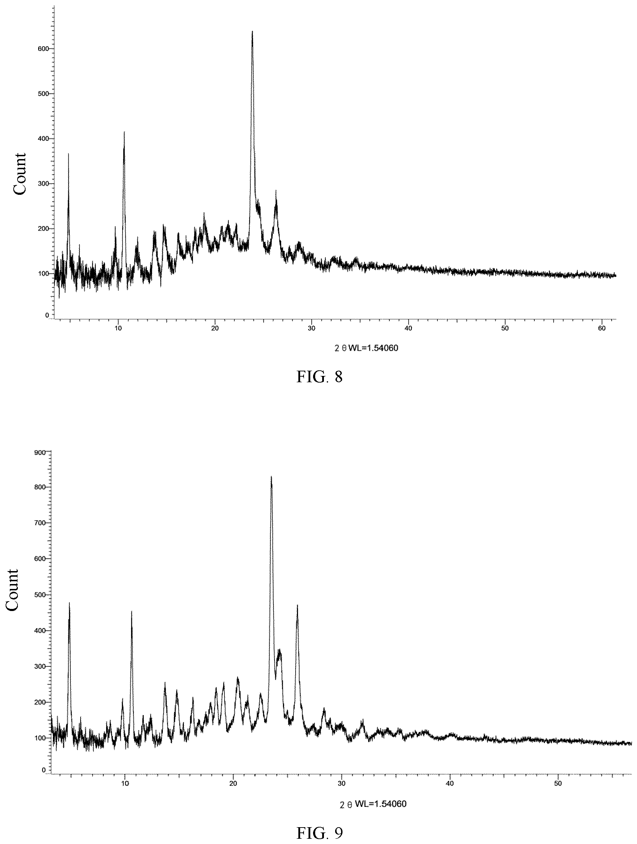

The amorphous compound of formula (I) (100 mg, 222.28 µmol) was added to 5 mL of acetonitrile, and 27.0 mg of fumaric acid was added. The mixture was stirred at 50 °C for 12-36 h and filtered under reduced pressure. The filter cake was dried *in vacuo* at 40 °C. The product was identified by X-ray powder diffraction as crystal form III. The XRPD graph is shown in FIG. 8, and the peak positions are shown in Table 7. NMR analysis shows that the molar ratio of the compound of formula (I) to fumaric acid in the salt is 2:1.

**Table 7. The peak positions of the fumarate in crystal form III**

| Peak number | 2θ(°) | d(Å) | I(%) |
|---|---|---|---|
| 1 | 4.877 | 18.10485 | 47.10% |
| 2 | 9.664 | 9.14464 | 14.40% |
| 3 | 10.601 | 8.33869 | 62.00% |
| 4 | 11.745 | 7.52842 | 12.10% |
| 5 | 13.827 | 6.39948 | 18.00% |
| 6 | 14.867 | 5.95380 | 17.70% |
| 7 | 16.272 | 5.44278 | 15.50% |
| 8 | 17.885 | 4.95538 | 8.50% |
| 9 | 18.874 | 4.69798 | 16.00% |
| 10 | 20.695 | 4.28849 | 6.90% |
| 11 | 21.320 | 4.16428 | 8.00% |
| 12 | 22.204 | 4.00034 | 8.90% |
| 13 | 23.921 | 3.71693 | 100.00% |
| 14 | 26.367 | 3.37746 | 25.80% |

### Example 5: Preparation of Fumarate in Crystal Form IV

A 100-mg sample of the fumarate in crystal form III was left to stand at 93% RH overnight. The product was identified by X-ray powder diffraction as crystal form IV The XRPD graph is shown in FIG. 9, and the peak positions are shown in Table 8. The TGA graph shows that the crystal form made a weight loss of 0.73% at 30-100 °C and a weight loss of 10.89% at 120-200 °C. The DSC graph shows that the crystal form has an endothermic peak at 148.3 °C. NMR analysis shows that the molar ratio of the compound of formula (I) to fumaric acid in the salt is 2:1.

**Table 8. The peak positions of the fumarate in crystal form IV**

| Peak number | 2θ(°) | d(Å) | I(%) |
|---|---|---|---|
| 1 | 4.854 | 18.18903 | 57.10% |
| 2 | 8.650 | 10.21482 | 10.10% |
| 3 | 9.785 | 9.03188 | 18.40% |
| 4 | 10.613 | 8.32887 | 48.70% |
| 5 | 11.658 | 7.58494 | 11.20% |
| 6 | 12.449 | 7.10432 | 10.60% |
| 7 | 13.692 | 6.46226 | 23.70% |
| 8 | 14.794 | 5.98320 | 19.50% |
| 9 | 15.393 | 5.75165 | 5.50% |
| 10 | 16.257 | 5.44796 | 15.00% |
| 11 | 17.864 | 4.96136 | 9.90% |
| 12 | 18.424 | 4.81168 | 16.30% |
| 13 | 19.107 | 4.64128 | 18.00% |
| 14 | 20.409 | 4.34794 | 19.50% |
| 15 | 21.322 | 4.16387 | 9.40% |
| 16 | 22.510 | 3.94672 | 12.10% |
| 17 | 23.523 | 3.77893 | 100.00% |
| 18 | 24.252 | 3.66707 | 27.50% |
| 19 | 25.925 | 3.43408 | 49.80% |
| 20 | 28.382 | 3.14209 | 10.50% |

### Example 6: Preparation of Oxalate in Crystal Form a

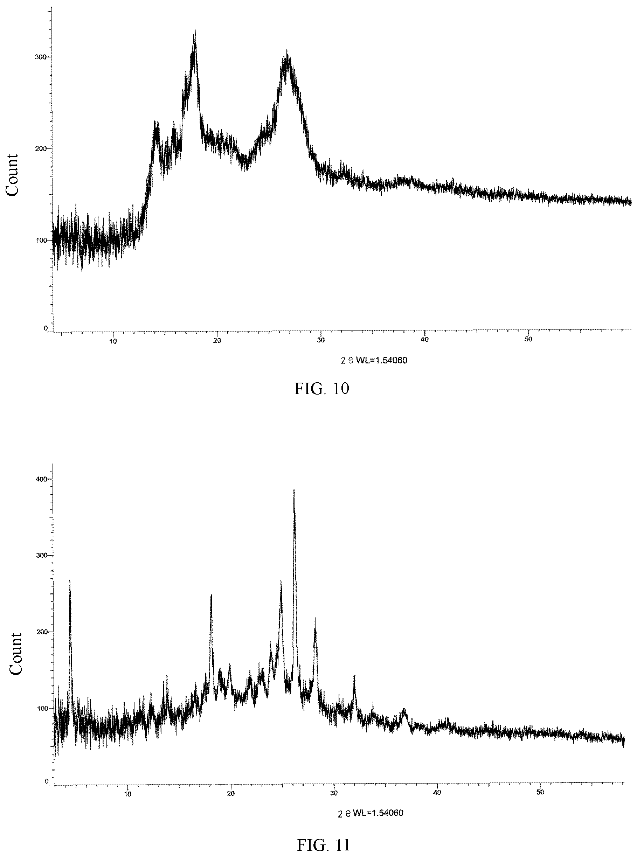

100 mg of the amorphous compound of formula (I) was measured out, and 10.5 mg of oxalic acid and 5 mL of n-hexane were added. The mixture was stirred at 50 °C for 24-36 h and filtered under reduced pressure. The filter cake was dried *in vacuo* at 40 °C to give a solid. The product was identified by X-ray powder diffraction as crystal form a. The XRPD graph is shown in FIG. 10, and the peak positions are shown in Table 9.

**Table 9. The peak positions of the oxalate in crystal form a**

| Peak number | 2θ(°) | d(Å) | I(%) |
|---|---|---|---|
| 1 | 14.235 | 6.21682 | 69.60% |
| 2 | 15.983 | 5.54054 | 36.60% |
| 3 | 17.882 | 4.95646 | 100.00% |
| 4 | 26.923 | 3.30901 | 89.70% |

### Example 7: Preparation of Maleate in Crystal Form a

The amorphous compound of formula (I) (100 mg, 222.28 µmol) was added to 2 mL of isopropanol. The temperature was raised to 60 °C, and 29 mg of maleic acid was added.

The mixture was stirred at 60 °C for 30 min, cooled to room temperature, and stirred at room temperature for 2 h. A small amount of solid gradually precipitated. The stirring continued at room temperature overnight, and more solid precipitated. The mixture was filtered under reduced pressure, and the filter cake was collected, washed with a small amount of isopropanol, and dried *in vacuo* at 40 °C to give a product (90.1 mg). The ¹H-NMR analysis of the product is shown below and indicates that the molar ratio of the compound of formula (I) to maleic acid in the salt is 1:0.95.
¹H NMR (400 MHz, DMSO- *d₆*): 9.33 (s,1H) 8.37 (d, 1H), 7.92 (s, 1H), 7.48 (d, 1H), 7.41-7.33 (m, 3H), 7.28-7.24 (m, 1H), 7.18 (s, 1H), 6.66 (s, 1H), 6.27 (s, 2H), 5.17 (dd, 1H), 4.66 (d, 1H), 4.42 (dd, 1H), 3.94-4.06 (m, 2H), 3.69 (s, 3H).

The product was identified by X-ray powder diffraction as crystal form a. The XRPD graph is shown in FIG. 11, and the peak positions are shown in Table 10.

**Table 10. The peak positions of the maleate in crystal form a**

| Peak number | 2θ(°) | d(Å) | I(%) |
|---|---|---|---|
| 1 | 4.510 | 19.5785 | 70.90% |
| 2 | 18.170 | 4.87846 | 54.00% |
| 3 | 19.959 | 4.44501 | 17.10% |
| 4 | 21.842 | 4.06581 | 8.80% |
| 5 | 23.973 | 3.70899 | 22.10% |
| 6 | 24.864 | 3.57811 | 42.40% |
| 7 | 26.306 | 3.38517 | 100.00% |
| 8 | 28.209 | 3.16096 | 39.90% |
| 9 | 31.995 | 2.79505 | 18.40% |

### Example 8: Preparation of Maleate in Crystal Form b

The amorphous compound of formula (I) (100 mg, 222.28 µmol) was added to 2 mL of ethyl acetate. The temperature was raised to 60 °C, and 29 mg of maleic acid was added. The mixture was stirred at 60 °C for 30 min, cooled to room temperature, and stirred at room temperature for 2 h. A solid gradually precipitated. The stirring continued at room temperature overnight. The mixture was filtered under reduced pressure, and the filter cake was collected, washed with a small amount of ethyl acetate, and dried *in vacuo* at 40 °C to give a product (125.1 mg).

The ¹H-NMR analysis of the product is shown below and indicates that the molar ratio of the compound of formula (I) to maleic acid in the salt is 1:0.97.
1H NMR (400 MHz, DMSO- *d₆*): 9.33 (s,1H), 8.37 (d, 1H), 7.92 (s, 1H), 7.48 (d, 1H), 7.41-7.33 (m, 3H), 7.28-7.24 (m, 1H), 7.18 (s, 1H), 6.66 (s, 1H), 6.27 (s, 2H), 5.17 (dd, 1H), 4.66 (d, 1H), 4.42 (dd, 1H), 3.94-4.06 (m, 2H), 3.69 (s, 3H).

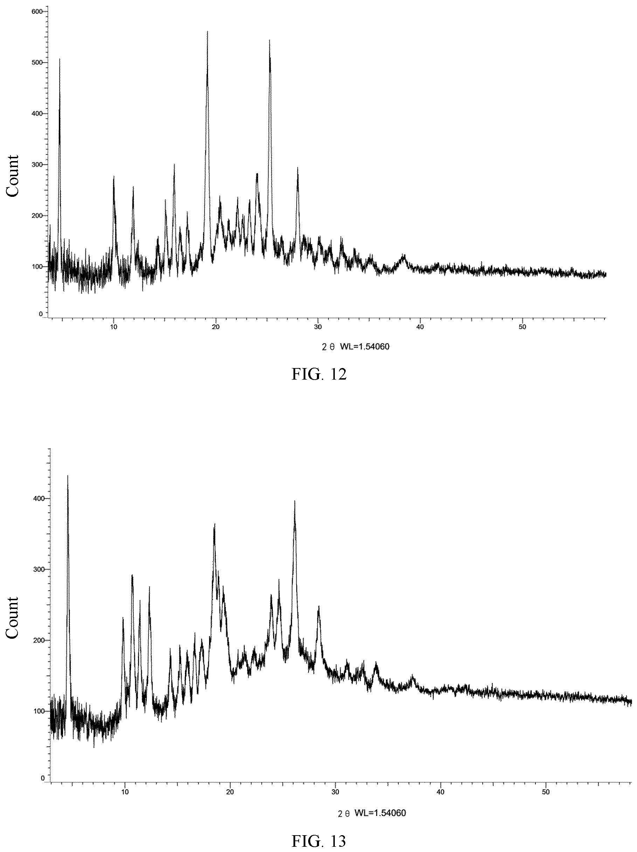

The product was identified by X-ray powder diffraction as crystal form b. The XRPD graph is shown in FIG. 12, and the peak positions are shown in Table 11.

**Table 11. The peak positions of the maleate in crystal form b**

| Peak number | 2θ(°) | d(Å) | I(%) |
|---|---|---|---|
| 1 | 4.744 | 18.61048 | 100.00% |
| 2 | 10.024 | 8.81670 | 44.50% |
| 3 | 11.931 | 7.41168 | 42.80% |
| 4 | 14.420 | 6.13735 | 10.30% |
| 5 | 15.185 | 5.82993 | 25.90% |
| 6 | 15.969 | 5.54536 | 39.60% |
| 7 | 17.249 | 5.13667 | 24.20% |
| 8 | 19.171 | 4.62586 | 77.40% |
| 9 | 20.405 | 4.34883 | 20.10% |
| 10 | 24.110 | 3.68827 | 29.90% |
| 11 | 25.291 | 3.51869 | 83.10% |
| 12 | 28.006 | 3.18345 | 38.40% |
| 13 | 30.216 | 2.95538 | 7.20% |
| 14 | 32.380 | 2.76265 | 10.10% |

### Example 9: Preparation of Maleate in Crystal Form c

The amorphous compound of formula (I) (100 mg) was added to 13.5 mg of maleic acid and 2 mL of acetonitrile. The mixture was stirred at 50 °C for 3 h and filtered under reduced pressure. The filter cake was dried *in vacuo* at 40 °C to give a solid. The ¹H-NMR analysis of the product is shown below and indicates that the molar ratio of the compound of formula (I) to maleic acid in the salt is 1:1.
¹H NMR (400 MHz, DMSO- *d₆*): 9.33 (s,1H), 8.37 (d, 1H), 7.92 (s, 1H), 7.48 (d, 1H), 7.41-7.33 (m, 3H), 7.28-7.24 (m, 1H), 7.18 (s, 1H), 6.66 (s, 1H), 6.27 (s, 2H), 5.17 (dd, 1H), 4.66 (d, 1H), 4.42 (dd, 1H), 3.94-4.06 (m, 2H), 3.69 (s, 3H)

The product was identified by X-ray powder diffraction as crystal form c. The XRPD graph is shown in FIG. 13, and the peak positions are shown in Table 12.

**Table 12. The peak positions of the maleate in crystal form c**

| Peak number | 2θ(°) | d(Å) | I(%) |
|---|---|---|---|
| 1 | 4.610 | 19.15107 | 100.00% |
| 2 | 9.840 | 8.98151 | 44.20% |
| 3 | 10.708 | 8.25568 | 63.20% |
| 4 | 11.414 | 7.74636 | 45.80% |
| 5 | 12.329 | 7.17343 | 54.10% |
| 6 | 12.395 | 7.13547 | 42.70% |
| 7 | 14.346 | 6.16912 | 20.40% |
| 8 | 15.262 | 5.80091 | 25.20% |
| 9 | 15.962 | 5.54808 | 16.20% |
| 10 | 16.655 | 5.31869 | 22.60% |
| 11 | 17.298 | 5.12221 | 20.20% |
| 12 | 18.485 | 4.79590 | 61.10% |
| 13 | 18.883 | 4.69577 | 42.90% |
| 14 | 19.328 | 4.58858 | 42.50% |
| 15 | 22.250 | 3.99230 | 5.60% |
| 16 | 23.928 | 3.71594 | 23.80% |
| 17 | 26.160 | 3.40370 | 69.20% |
| 18 | 28.389 | 3.14134 | 23.80% |
| 19 | 31.162 | 2.86786 | 4.20% |

### Example 10: Preparation of Maleate in Amorphous Form

The free base of the compound of formula (I) (500 mg, 1.11 mmol) was added to 10 mL of methanol, and 129 mg of maleic acid was added. The mixture was heated to 60 °C, stirred for 30 min, and concentrated by rotary evaporation to give the title product (605 mg, yield: 96.2%).

The ¹H-NMR analysis of the product is shown below and indicates that the molar ratio of the compound of formula (I) to maleic acid in the salt is 1:1.
¹H NMR (400 MHz, DMSO- *d₆*): 9.33 (s,1H) 8.37 (d, 1H), 7.92 (s, 1H), 7.48 (d, 1H), 7.41-7.33 (m, 3H), 7.28-7.24 (m, 1H), 7.18 (s, 1H), 6.66 (s, 1H), 6.27 (s, 2H), 5.17 (dd, 1H), 4.66 (d, 1H), 4.42 (dd, 1H), 3.94-4.06 (m, 2H), 3.69 (s, 3H)

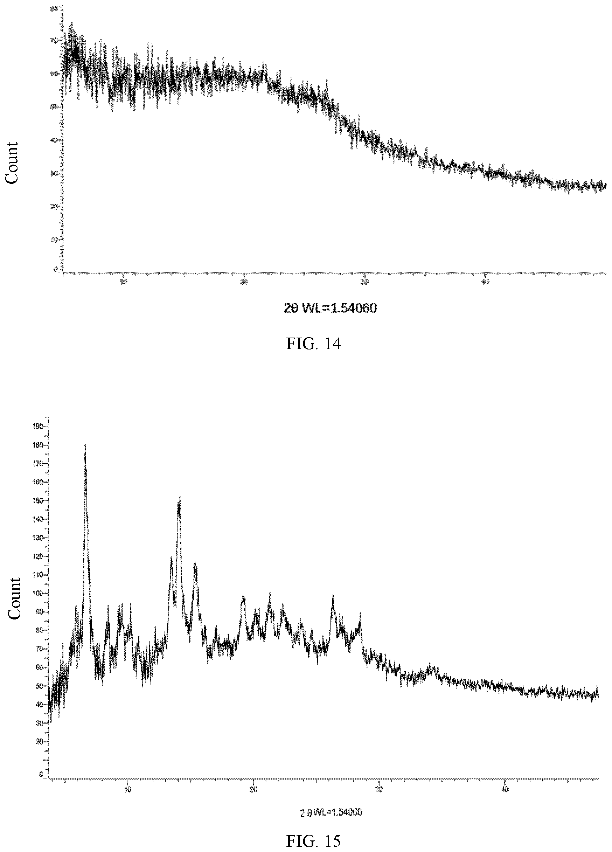

The product was identified by X-ray powder diffraction as amorphous. The XRPD graph is shown in FIG. 14.

### Example 11: Preparation of Maleate in Crystal Form d

The amorphous maleate of the compound of formula (I) (Example 10) (36 mg, 63.6 µmol) was added to 0.4 mL of toluene and triturated at room temperature for 72 h. The mixture was filtered, and the filter cake was collected and dried *in vacuo* at 40 °C to give the title product (26.0 mg, yield: 73.4%).

The ¹H-NMR analysis of the product is shown below and indicates that the molar ratio of the compound of formula (I) to maleic acid in the salt is 1:1.
1H NMR (400 MHz, DMSO- *d₆*): 9.31 (s,1H) 8.35 (d, 1H), 7.91 (s, 1H), 7.48 (d, 1H), 7.41-7.33 (m, 3H), 7.28-7.24 (m, 1H), 7.15 (s, 1H), 6.63 (s, 1H), 6.27 (s, 2H), 5.17 (dd, 1H), 4.63 (d, 1H), 4.42 (dd, 1H), 3.94-4.06 (m, 2H), 3.71 (s, 3H).

The product was identified by X-ray powder diffraction as crystal form d. The XRPD graph is shown in FIG. 15, and the peak positions are shown in Table 13.

**Table 13. The peak positions of the maleate in crystal form d**

| Peak number | 2θ(°) | d(Å) | I(%) |
|---|---|---|---|
| 1 | 6.679 | 13.22322 | 100.0% |
| 2 | 8.405 | 10.51102 | 19.70% |
| 3 | 9.346 | 9.45469 | 23.30% |
| 4 | 10.170 | 8.69093 | 21.10% |
| 5 | 10.876 | 8.12845 | 15.10% |
| 6 | 13.476 | 6.56514 | 47.70% |
| 7 | 14.076 | 6.28693 | 71.20% |
| 8 | 15.388 | 5.75338 | 41.60% |
| 9 | 16.993 | 5.21367 | 9.00% |
| 10 | 19.183 | 4.62300 | 22.60% |
| 11 | 20.286 | 4.37398 | 7.10% |
| 12 | 21.228 | 4.18215 | 10.50% |
| 13 | 22.286 | 3.98581 | 9.70% |
| 14 | 23.894 | 3.72115 | 10.70% |
| 15 | 24.639 | 3.61029 | 11.70% |
| 16 | 26.247 | 3.39268 | 18.10% |
| 17 | 27.070 | 3.29132 | 11.80% |
| 18 | 28.403 | 3.13979 | 12.20% |

### Example 12: Preparation of Maleate in Crystal Form e

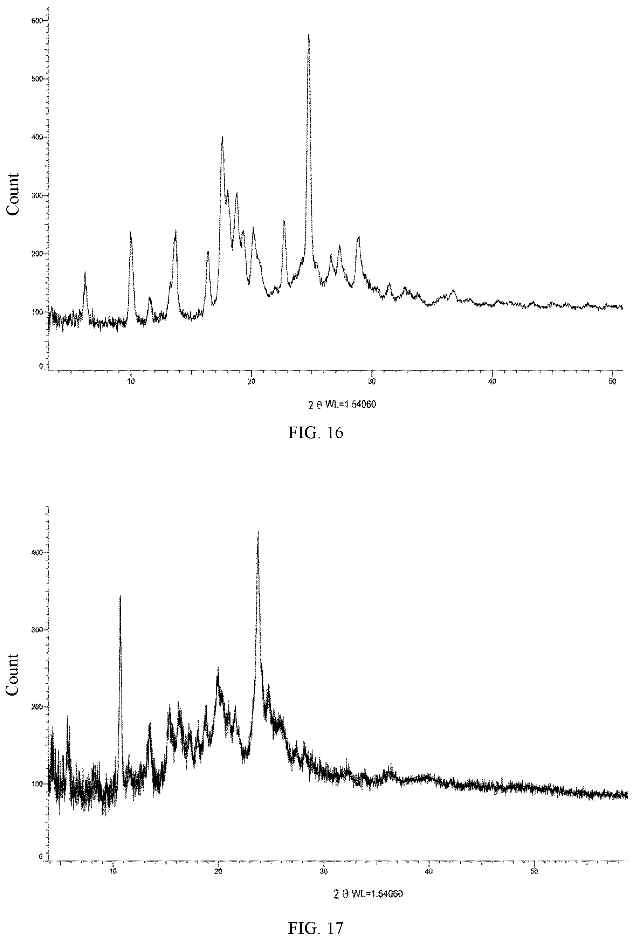

The maleate in crystal form c was dried at 60 °C overnight to give a solid. The product was identified by X-ray powder diffraction as crystal form e. The XRPD graph is shown in FIG. 16, and the peak positions are shown in Table 14.

**Table 14. The peak positions of the maleate in crystal form e**

| Peak number | 2θ(°) | d(Å) | I(%) |
|---|---|---|---|
| 1 | 6.193 | 14.25955 | 20.10% |
| 2 | 10.009 | 8.83064 | 34.40% |
| 3 | 11.591 | 7.62821 | 10.10% |
| 4 | 13.646 | 6.48371 | 32.50% |
| 5 | 16.375 | 5.40878 | 23.60% |
| 6 | 17.582 | 5.04031 | 66.00% |
| 7 | 17.996 | 4.92509 | 40.70% |
| 8 | 18.758 | 4.72669 | 39.20% |
| 9 | 19.306 | 4.59395 | 24.50% |
| 10 | 20.181 | 4.39656 | 24.60% |
| 11 | 22.733 | 3.90842 | 26.70% |
| 12 | 24.766 | 3.59202 | 100.00% |
| 13 | 25.332 | 3.51301 | 6.60% |
| 14 | 26.601 | 3.34827 | 11.20% |
| 15 | 27.303 | 3.26381 | 15.10% |
| 16 | 28.859 | 3.09127 | 19.70% |
| 17 | 31.447 | 2.84248 | 5.10% |

### Example 13: Preparation of Succinate in Crystal Form α

100 mg of the free base of the compound of formula (I) was measured out, and 13.8 mg of succinic acid and 5 mL of isopropyl ether were added. The mixture was stirred at 50 °C for 12-36 h and filtered under reduced pressure. The filter cake was dried *in vacuo* at 40 °C. The product was identified as crystal form α. The XRPD graph is shown in FIG. 17, and the peak positions are shown in Table 15.

**Table 15. The peak positions of the succinate in crystal form α**

| Peak number | 2θ(°) | d(Å) | I(%) |
|---|---|---|---|
| 1 | 5.640 | 15.65567 | 28.80% |
| 2 | 10.720 | 8.24589 | 84.20% |
| 3 | 13.384 | 6.61041 | 27.10% |
| 4 | 15.208 | 5.82111 | 28.20% |
| 5 | 16.244 | 5.45222 | 24.50% |
| 6 | 17.280 | 5.12769 | 11.70% |
| 7 | 18.020 | 4.91881 | 9.00% |
| 8 | 18.907 | 4.68981 | 10.20% |
| 9 | 19.992 | 4.43767 | 42.00% |
| 10 | 21.669 | 4.09791 | 17.80% |
| 11 | 23.839 | 3.72956 | 100.00% |
| 12 | 24.826 | 3.58357 | 33.10% |

### Example 14: Preparation of Succinate in Crystal Form β

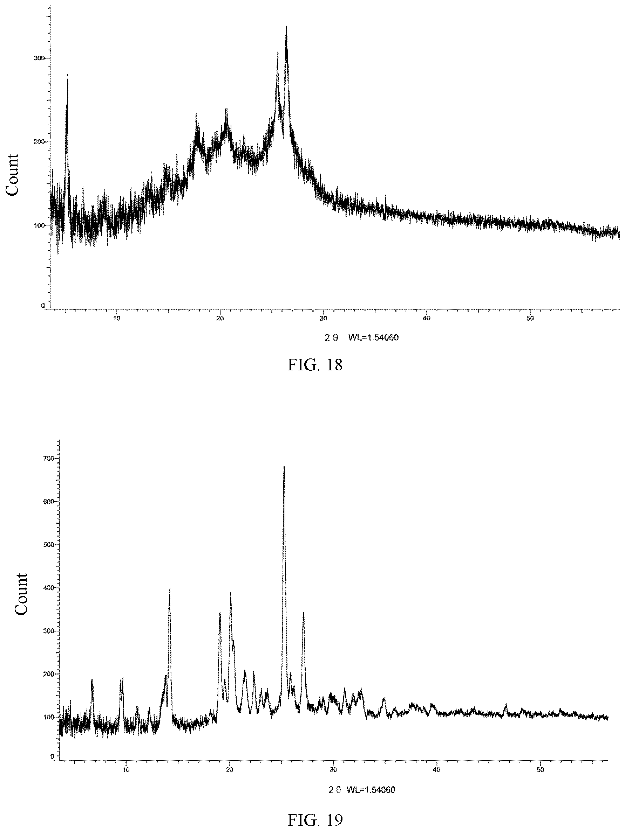

100 mg of the free base of the compound of formula (I) was measured out, and 13.8 mg of succinic acid and 5 mL of acetonitrile were added. The mixture was stirred at 50 °C for 12-36 h and filtered under reduced pressure. The filter cake was dried *in vacuo* at 40 °C. The product was identified as crystal form β. The XRPD graph is shown in FIG. 18, and the peak positions are shown in Table 16.

**Table 16. The peak positions of the succinate in crystal form β**

| Peak number | 2θ(°) | d(Å) | I(%) |
|---|---|---|---|
| 1 | 5.160 | 17.11116 | 100.00% |
| 2 | 17.951 | 4.93733 | 23.90% |
| 3 | 20.618 | 4.30434 | 17.40% |
| 4 | 25.557 | 3.48266 | 68.70% |
| 5 | 26.545 | 3.35527 | 75.90% |

### Example 15: Preparation of Hydrobromide in Crystal Form α

100 mg of the free base of the compound of formula (I) was measured out, and 7 µL of hydrobromic acid and 5 mL of acetonitrile were added. The mixture was stirred at 50 °C for 2-36 h and filtered under reduced pressure. The filter cake was dried *in vacuo* at 40 °C. Ion chromatography analysis shows that the bromide ion content of the product is 7.6% and the molar ratio of the compound of formula (I) to hydrobromic acid in the salt is 1: 1. The product was identified as crystal form α. The XRPD graph is shown in FIG. 19, and the peak positions are shown in Table 17. The TGA graph shows that the crystal form made a weight loss of 0.27% at 30-170 °C and a weight loss of 14.47% at 190-290 °C. The DSC graph shows that the crystal form has an endothermic peak at 175.85 °C.

**Table 17. The peak positions of the hydrobromide in crystal form α**

| Peak number | 2θ(°) | d(Å) | I(%) |
|---|---|---|---|
| 1 | 6.656 | 13.26907 | 20.20% |
| 2 | 9.552 | 9.25152 | 14.60% |
| 3 | 13.778 | 6.42219 | 19.50% |
| 4 | 14.110 | 6.27166 | 49.90% |
| 5 | 19.095 | 4.64409 | 43.20% |
| 6 | 19.522 | 4.54340 | 14.50% |
| 7 | 20.092 | 4.41583 | 46.40% |
| 8 | 21.422 | 4.14470 | 12.30% |
| 9 | 22.276 | 3.98759 | 10.60% |
| 10 | 22.988 | 3.86564 | 6.70% |
| 11 | 23.653 | 3.75850 | 9.70% |
| 12 | 25.220 | 3.52844 | 100.00% |
| 13 | 25.837 | 3.44553 | 14.50% |
| 14 | 26.217 | 3.39647 | 7.60% |
| 15 | 27.119 | 3.28551 | 40.40% |
| 16 | 31.107 | 2.87277 | 8.60% |
| 17 | 31.867 | 2.80600 | 6.90% |
| 18 | 32.769 | 2.73079 | 8.20% |
| 19 | 34.810 | 2.57517 | 6.40% |

### Example 16: Preparation of Hydrobromide in Crystal Form β

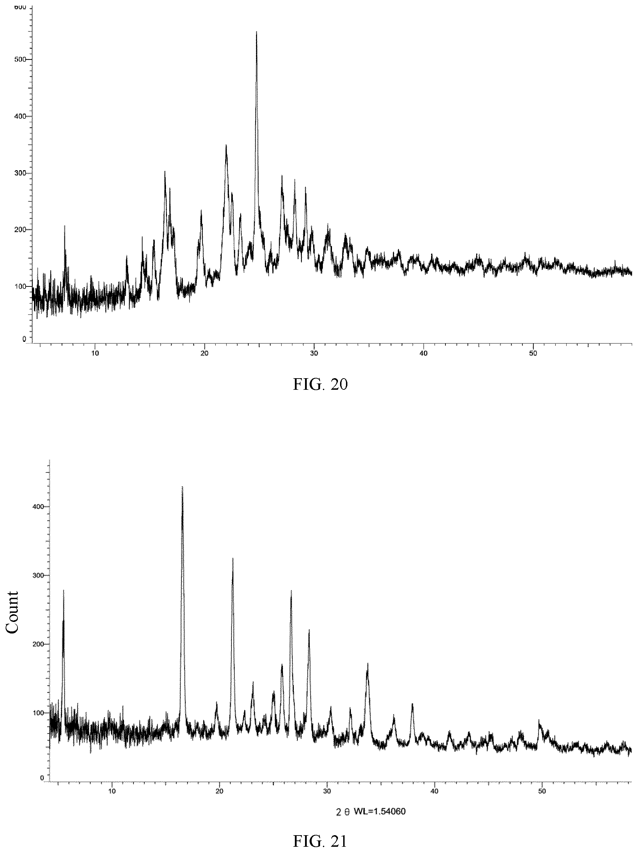

100 mg of the free base of the compound of formula (I) was measured out, and 14 µL of hydrobromic acid and 5 mL of acetonitrile were added. The mixture was stirred at 50 °C for 2 h and filtered under reduced pressure. The filter cake was dried *in vacuo* at 40 °C. Ion chromatography analysis shows that the bromide ion content of the product is 14.3% and the molar ratio of the compound of formula (I) to hydrobromic acid in the salt is 1:2. The product was identified as crystal form β. The XRPD graph is shown in FIG. 20, and the peak positions are shown in Table 18. The TGA graph shows that the crystal form made a weight loss of 0.58% at 30-130 °C and a weight loss of 17.26% at 150-290 °C. The DSC graph shows that the crystal form has an endothermic peak at 213.86 °C.

**Table 18. The peak positions of the hydrobromide in crystal form β**

| Peak number | 2θ(°) | d(Å) | I(%) |
|---|---|---|---|
| 1 | 7.363 | 11.99692 | 23.00% |
| 2 | 12.914 | 6.84991 | 21.30% |
| 3 | 14.351 | 6.16695 | 17.90% |
| 4 | 15.392 | 5.75220 | 23.60% |
| 5 | 16.865 | 5.25297 | 40.50% |
| 6 | 17.217 | 5.14629 | 28.60% |
| 7 | 19.452 | 4.55974 | 19.40% |
| 8 | 19.704 | 4.50185 | 32.40% |
| 9 | 21.999 | 4.03719 | 51.50% |
| 10 | 22.515 | 3.94590 | 31.70% |
| 11 | 23.275 | 3.81865 | 22.60% |
| 12 | 24.213 | 3.67275 | 8.60% |
| 13 | 24.769 | 3.59161 | 100.00% |
| 14 | 27.056 | 3.29296 | 36.40% |
| 15 | 28.236 | 3.15797 | 32.70% |
| 16 | 29.234 | 3.05243 | 26.80% |
| 17 | 29.764 | 2.99924 | 15.00% |
| 18 | 31.251 | 2.85985 | 19.60% |
| 19 | 32.887 | 2.72127 | 9.30% |

### Example 17: Preparation of Hydrobromide in Crystal Form γ

100 mg of the free base of the compound of formula (I) was measured out, and 14 µL of hydrobromic acid and 5 mL of acetonitrile were added. The mixture was stirred at 50 °C for 36 h and filtered under reduced pressure. The filter cake was dried *in vacuo* at 40 °C for 1 h. The product was identified as crystal form γ. The XRPD graph is shown in FIG. 21, and the peak positions are shown in Table 19.

**Table 19. The peak positions of the hydrobromide in crystal form γ**

| Peak number | 2θ(°) | d(Å) | I(%) |
|---|---|---|---|
| 1 | 5.463 | 16.16374 | 41.70% |
| 2 | 16.567 | 5.34670 | 100.00% |
| 3 | 19.733 | 4.49534 | 9.50% |
| 4 | 21.227 | 4.18229 | 66.70% |
| 5 | 22.332 | 3.97776 | 7.70% |
| 6 | 25.014 | 3.55697 | 14.10% |
| 7 | 25.784 | 3.45244 | 28.00% |
| 8 | 26.643 | 3.34307 | 56.10% |
| 9 | 28.321 | 3.14868 | 38.50% |
| 10 | 30.298 | 2.94759 | 11.60% |
| 11 | 32.170 | 2.78026 | 12.50% |
| 12 | 33.788 | 2.65074 | 30.00% |
| 13 | 36.211 | 2.47872 | 8.10% |
| 14 | 37.939 | 2.36970 | 14.30% |

### Example 18: Preparation of Hydrochloride in Crystal Form I

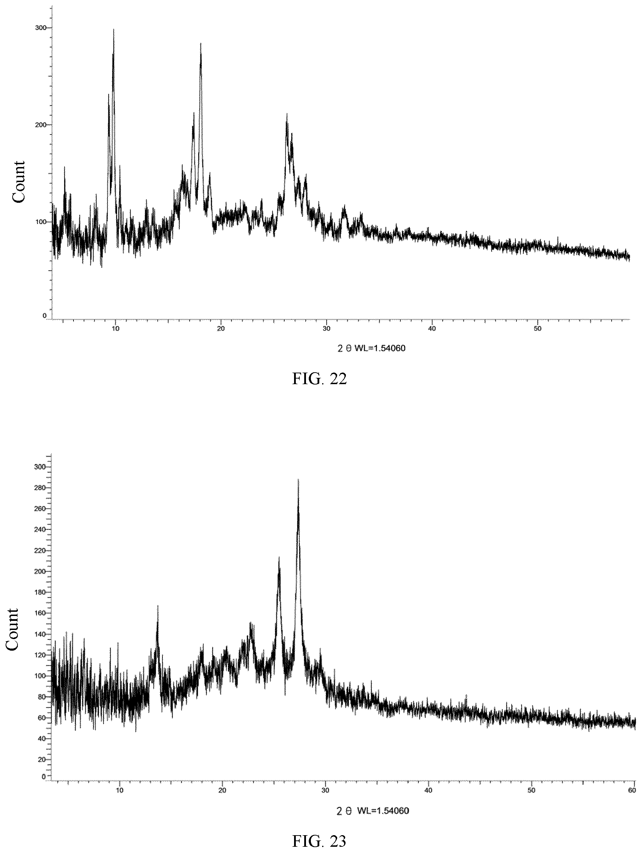

100 mg of the free base of the compound of formula (I) was measured out, and 20 µL of hydrochloric acid and 5 mL of acetonitrile were added. The mixture was stirred at 50 °C for 36 h and filtered under reduced pressure. The filter cake was dried *in vacuo* at 40 °C. Ion chromatography analysis shows that the chloride ion content of the product is 6.52%, which indicates that the molar ratio of the compound of formula (I) to hydrochloric acid in the salt is 1:1. The product was identified as crystal form I. The XRPD graph is shown in FIG. 22, and the peak positions are shown in Table 20.

**Table 20. The peak positions of the hydrochloride in crystal form I**

| Peak number | 2θ(°) | d(Å) | I(%) |
|---|---|---|---|
| 1 | 5.187 | 17.02196 | 26.50% |
| 2 | 8.131 | 10.86513 | 15.70% |
| 3 | 9.407 | 9.39446 | 55.90% |
| 4 | 9.848 | 8.97424 | 100.00% |
| 5 | 10.388 | 8.50920 | 33.80% |
| 6 | 17.354 | 5.10590 | 42.80% |
| 7 | 18.041 | 4.91303 | 67.20% |
| 8 | 18.924 | 4.68572 | 18.90% |
| 9 | 23.830 | 3.73100 | 11.40% |
| 10 | 25.449 | 3.49720 | 8.70% |
| 11 | 26.234 | 3.39431 | 42.80% |
| 12 | 26.724 | 3.33310 | 31.80% |
| 13 | 27.362 | 3.25684 | 19.80% |
| 14 | 27.902 | 3.19507 | 16.10% |
| 15 | 29.275 | 3.04820 | 10.50% |
| 16 | 31.581 | 2.83071 | 6.90% |

### Example 19: Preparation of Hydrochloride in Crystal Form II

100 mg of the free base of the compound of formula (I) was measured out, and 20 µL of hydrochloric acid and 5 mL of ethanol were added. The mixture was made clear by being stirred at 50 °C for 1 h, and was cooled at room temperature for 1 h, stirred at 25 °C for 36 h, and filtered under reduced pressure. The filter cake was dried *in vacuo* at 40 °C. Ion chromatography analysis shows that the chloride ion content of the product is 6.88%, which indicates that the molar ratio of the compound of formula (I) to hydrochloric acid in the salt is 1:1. The product was identified as crystal form II. The XRPD graph is shown in FIG. 23, and the peak positions are shown in Table 21.

**Table 21. The peak positions of the hydrochloride in crystal form II**

| Peak number | 2θ(°) | d(Å) | I(%) |
|---|---|---|---|
| 1 | 13.728 | 6.44541 | 49.10% |
| 2 | 17.948 | 4.93813 | 17.30% |
| 3 | 20.593 | 4.30962 | 6.90% |
| 4 | 22.678 | 3.9179 | 18.10% |
| 5 | 25.525 | 3.4869 | 53.70% |
| 6 | 27.356 | 3.25757 | 100.00% |
| 7 | 29.543 | 3.02125 | 13.20% |

### Example 20: Preparation of Hydrochloride in Crystal Form III

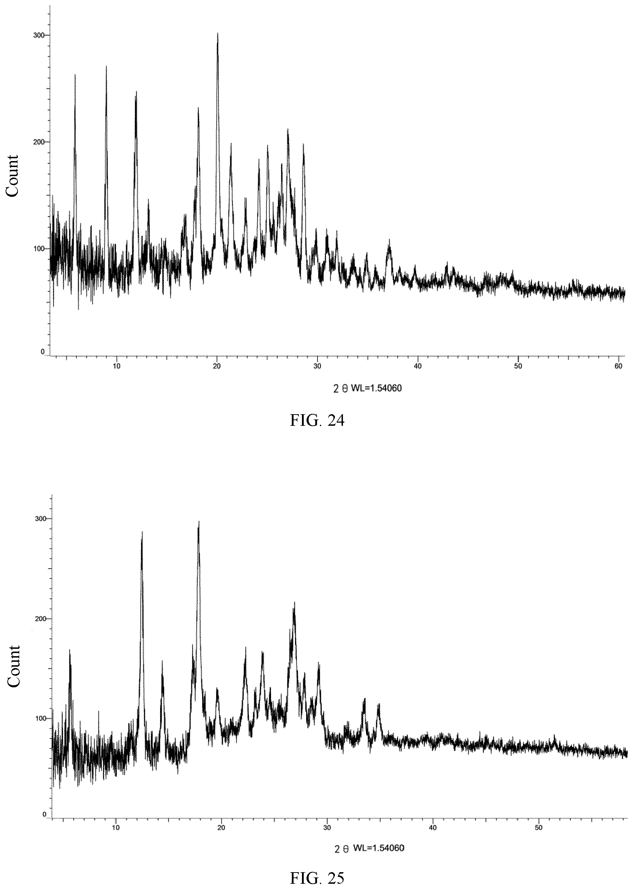

100 mg of the free base of the compound of formula (I) was measured out, and 10 µL of hydrochloric acid and 5 mL of ethanol were added. The mixture was made clear by being stirred at 50 °C for 1 h, and was cooled at room temperature for 1 h, stirred at 25 °C for 36 h, and filtered under reduced pressure. The filter cake was dried *in vacuo* at 40 °C. Ion chromatography analysis shows that the chloride ion content of the product is 6.82%, which indicates that the molar ratio of the compound of formula (I) to hydrochloric acid in the salt is 1:1. The product was identified as crystal form III. The XRPD graph is shown in FIG. 24, and the peak positions are shown in Table 22.

**Table 22. The peak positions of the hydrochloride in crystal form III**

| Peak number | 2θ(°) | d(Å) | I(%) |
|---|---|---|---|
| 1 | 5.854 | 15.08568 | 86.60% |
| 2 | 8.983 | 9.83680 | 93.70% |
| 3 | 11.902 | 7.42951 | 72.70% |
| 4 | 13.181 | 6.71157 | 36.30% |
| 5 | 16.876 | 5.24937 | 28.10% |
| 6 | 18.159 | 4.88131 | 68.30% |
| 7 | 20.071 | 4.42037 | 100.00% |
| 8 | 21.371 | 4.15444 | 47.30% |
| 9 | 22.799 | 3.89724 | 20.30% |
| 10 | 24.180 | 3.67779 | 43.60% |
| 11 | 25.066 | 3.54969 | 48.30% |
| 12 | 26.469 | 3.36471 | 36.10% |
| 13 | 27.086 | 3.28943 | 53.30% |
| 14 | 27.729 | 3.21454 | 33.70% |
| 15 | 28.615 | 3.11703 | 51.90% |
| 16 | 29.827 | 2.99309 | 15.20% |
| 17 | 30.904 | 2.89118 | 17.50% |
| 18 | 31.935 | 2.80013 | 18.00% |
| 19 | 33.571 | 2.66731 | 9.70% |
| 20 | 34.905 | 2.56839 | 11.60% |
| 21 | 37.265 | 2.41101 | 12.10% |

### Example 21: Preparation of Hydrochloride in Crystal Form IV

The hydrochloride in crystal form I was heated to 135 °C (10 K/min). The product was identified as crystal form IV The XRPD graph is shown in FIG. 25, and the peak positions are shown in Table 23.

**Table 23. The peak positions of the hydrochloride in crystal form IV**

| Peak number | 2θ(°) | d(Å) | I(%) |
|---|---|---|---|
| 1 | 5.693 | 15.51271 | 51.70% |
| 2 | 12.474 | 7.09045 | 100.00% |
| 3 | 14.474 | 6.11478 | 37.20% |
| 4 | 17.352 | 5.10641 | 38.80% |
| 5 | 17.840 | 4.96786 | 92.80% |
| 6 | 19.596 | 4.52641 | 18.00% |
| 7 | 22.231 | 3.99561 | 29.50% |
| 8 | 23.109 | 3.84572 | 15.70% |
| 9 | 23.938 | 3.71433 | 29.70% |
| 10 | 24.573 | 3.61988 | 12.50% |
| 11 | 26.866 | 3.31591 | 51.00% |
| 12 | 27.841 | 3.20188 | 21.70% |
| 13 | 29.207 | 3.05516 | 25.80% |
| 14 | 33.452 | 2.67658 | 14.10% |
| 15 | 34.915 | 2.56767 | 12.20% |

### Example 22: Preparation of Hydrochloride in Crystal Form V

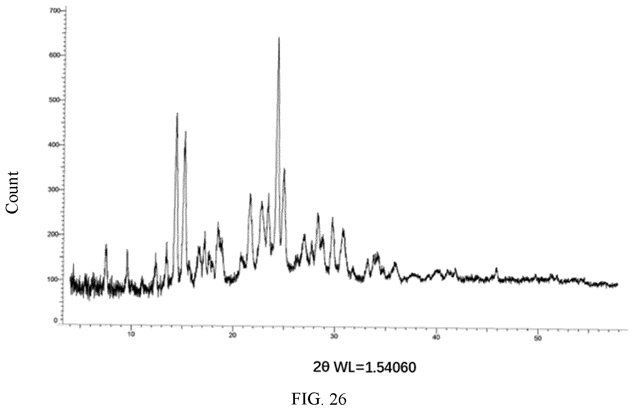

100 mg of the free base of the compound of formula (I) was measured out, and 40 µL of hydrochloric acid and 10 mL of ethanol were added. The mixture was made clear by being stirred at 50 °C for 1 h, and was stirred at 25 °C for 3-12 h and filtered under reduced pressure. The filter cake was dried *in vacuo* at 40 °C. The product was identified as crystal form V The XRPD graph is shown in FIG. 26, and the peak positions are shown in Table 24.

**Table 24. The peak positions of the hydrochloride in crystal form V**

| Peak number | 2θ(°) | d(Å) | I(%) |
|---|---|---|---|
| 1 | 7.476 | 11.8152 | 23.9% |
| 2 | 9.536 | 9.26702 | 19.4% |
| 3 | 12.349 | 7.16162 | 16.6% |
| 4 | 13.447 | 6.57921 | 21.5% |
| 5 | 14.346 | 6.16915 | 74.0% |
| 6 | 15.181 | 5.83170 | 72.9% |
| 7 | 15.639 | 5.66169 | 11.6% |
| 8 | 16.694 | 5.30619 | 16.2% |
| 9 | 17.185 | 5.15564 | 17.3% |
| 10 | 17.658 | 5.01861 | 9.6% |
| 11 | 17.877 | 4.95782 | 6.1% |
| 12 | 18.422 | 4.81217 | 24.2% |
| 13 | 18.859 | 4.70175 | 19.9% |
| 14 | 20.769 | 4.27348 | 6.4% |
| 15 | 21.606 | 4.10983 | 35.5% |
| 16 | 22.770 | 3.90227 | 29.4% |
| 17 | 23.425 | 3.79464 | 30.4% |
| 18 | 24.279 | 3.66292 | 100.0% |
| 19 | 24.952 | 3.56563 | 41.2% |
| 20 | 26.239 | 3.39365 | 1.8% |
| 21 | 26.990 | 3.30093 | 11.3% |
| 22 | 27.754 | 3.21178 | 7.7% |
| 23 | 28.336 | 3.14711 | 23.1% |
| 24 | 28.772 | 3.10034 | 13.7% |
| 25 | 29.773 | 2.99840 | 20.9% |
| 26 | 30.846 | 2.89648 | 18.5% |
| 27 | 33.089 | 2.70507 | 5.2% |
| 28 | 34.183 | 2.62095 | 8.4% |
| 29 | 36.039 | 2.49016 | 4.4% |
| 30 | 39.987 | 2.25291 | 2.7% |

### Example 23. Influencing Factor Stability Study

The hydrobromide in crystal form α, the hydrobromide in crystal form β, the fumarate in crystal form II, and the fumarate in crystal form IV were left to stand open, and their stability under lighting conditions (4500 Lux), high-temperature conditions (40 °C and 60 °C), high-humidity conditions (75% RH and 92.5% RH) was investigated in a period of 30 days.

**Table 25. The influencing factor stability data of the hydrobromide in crystal form α**

| Conditions | Time (days) | Hydrobromide in crystal form α | | |
|---|---|---|---|---|
| | | Color and appearance | Main peak purity (%) | Crystal form |
| Initial | 0 | Off-white solid | 99.43 | Crystal form α |
| 40°C | 7 | Off-white solid | 99.42 | Did not change |
| | 14 | Off-white solid | 99.32 | Did not change |
| | 30 | Off-white solid | 99.28 | Did not change |
| 60°C | 7 | Off-white solid | 99.38 | Did not change |
| | 14 | Off-white solid | 98.99 | Did not change |
| | 30 | Off-white solid | 99.02 | Did not change |
| 75% RH | 7 | Off-white solid | 99.41 | Did not change |
| | 14 | Off-white solid | 99.30 | Did not change |
| | 30 | Off-white solid | 99.31 | Did not change |
| 92.5% RH | 7 | Off-white solid | 99.39 | Did not change |
| | 14 | Off-white solid | 99.42 | Did not change |
| | 30 | Off-white solid | 99.28 | Did not change |
| 4500 Lux | 7 | Off-white solid | 99.09 | Did not change |
| | 14 | Off-white solid | 98.94 | Did not change |
| | 30 | Off-white solid | 98.50 | Did not change |

**Table 26. The influencing factor stability data of the hydrobromide in crystal form β**

| Conditions | Time (days) | Hydrobromide in crystal form β | | |
|---|---|---|---|---|
| | | Color and appearance | Main peak purity (%) | Crystal form |
| Initial | 0 | Off-white solid | 99.12 | Crystal form β |
| 40°C | 7 | Off-white solid | 99.13 | Did not change |
| | 14 | Off-white solid | 99.07 | Did not change |
| | 30 | Off-white solid | 98.96 | Did not change |
| 60°C | 7 | Off-white solid | 98.82 | Did not change |
| | 14 | Off-white solid | 98.82 | Did not change |
| | 30 | Off-white solid | 98.88 | Did not change |
| 75% RH | 7 | Off-white solid | 99.13 | Did not change |
| | 14 | Off-white solid | 99.11 | Did not change |
| | 30 | Off-white solid | 99.11 | Did not change |
| 92.5% RH | 7 | Off-white solid | 99.09 | Did not change |
| | 14 | Off-white solid | 99.15 | Did not change |
| | 30 | Off-white solid | 98.93 | Did not change |
| 4500 Lux | 7 | Off-white solid | 98.99 | Did not change |
| | 14 | Off-white solid | 98.54 | Did not change |
| | 30 | Off-white solid | 98.28 | Did not change |

**Table 27. The results of the 30-day influencing factor study for the fumarate in crystal form II**

| Conditions | Time (days) | Fumarate in crystal form II | | |
|---|---|---|---|---|
| | | Color and appearance | Purity (%) | Weight gain (%) |
| Initial | 0 | White solid | 99.7 | |
| 4500 Lux | 5 | White solid | 99.6 | |
| | 10 | White solid | 99.6 | |
| | 30 | White solid | 98.4 | |
| 40°C | 5 | White solid | 99.6 | |
| | 10 | White solid | 99.5 | |
| | 30 | White solid | 99.2 | |
| 60°C | 5 | White solid | 99.5 | |
| | 10 | White solid | 99.5 | |
| | 30 | White solid | 98.0 | |
| RH 75% | 5 | White solid | 99.7 | 0.54 |
| | 10 | White solid | 99.7 | 1.03 |
| | 30 | White solid | 99.7 | 1.42 |
| RH 90% | 5 | White solid | 99.7 | 1.16 |
| | 10 | White solid | 99.7 | 2.77 |
| | 30 | White solid | 99.7 | 3.97 |

**Table 28. The influencing factor stability data of the fumarate in crystal form IV**

| Conditions | Time (days) | Fumarate in crystal form IV | | |
|---|---|---|---|---|
| | | Color and appearance | Main peak purity (%) | Crystal form |
| Initial | 0 | Pale yellow solid | 99.47 | Crystal form IV |
| 40°C | 7 | Pale yellow solid | 99.46 | Did not change |
| | 14 | Pale yellow solid | 99.47 | Did not change |
| | 30 | Pale yellow solid | 99.26 | Did not change |
| 60°C | 7 | Pale yellow solid | 99.45 | Did not change |
| | 14 | Pale yellow solid | 99.46 | Did not change |
| | 30 | Pale yellow solid | 99.23 | Did not change |
| 75% RH | 7 | Pale yellow solid | 99.52 | Did not change |
| | 14 | Pale yellow solid | 99.43 | Did not change |
| | 30 | Pale yellow solid | 99.36 | Did not change |
| 92.5% RH | 7 | Pale yellow solid | 99.47 | Did not change |
| | 14 | Pale yellow solid | 99.45 | Did not change |
| | 30 | Pale yellow solid | 99.40 | Did not change |
| 4500 Lux | 7 | Pale yellow solid | 99.43 | Did not change |
| | 14 | Pale yellow solid | 99.29 | Did not change |
| | 30 | Pale yellow solid | 99.19 | Did not change |

Conclusion: the influencing factor experiment shows that the hydrobromide in crystal form α, the hydrobromide in crystal form β, the fumarate in crystal form II, and the fumarate in crystal form IV have relatively good physical and chemical stability under high-temperature conditions (40 °C and 60 °C) and high-humidity conditions (75% and 92.5%).

### Example 24. Long-Term/Accelerated Stability Study

1. The hydrobromide in crystal form α was left to stand under 25 °C, 60% RH conditions and 40 °C, 75% RH conditions to investigate its stability.

**Table 29**

| | Conditions of sitting | Purity (%) | Purity (%) | Crystal form |
|---|---|---|---|---|
| | | Initial | 1 month | |
| Sample | 25°C, 60%RH | 99.43 | 99.33 | α |
| | 40°C, 75%RH | | 99.24 | α |

The long-term/accelerated stability study shows that the hydrobromide in crystal form α has good physical and chemical stability when left to stand under long-term and accelerated stability conditions for 1 month.
2. The hydrobromide in crystal form β was left to stand under 25 °C, 60% RH conditions and 40 °C, 75% RH conditions to investigate its stability.

**Table 30**

| | Conditions of sitting | Purity (%) | Purity (%) | Crystal form |
|---|---|---|---|---|
| | | Initial | 1 month | |
| Sample | 25°C, 60%RH | 99.17 | 98.99 | β |
| | 40°C, 75%RH | | 99.00 | β |

The long-term/accelerated stability study shows that the hydrobromide in crystal form β has good physical and chemical stability when left to stand under long-term and accelerated stability conditions for 1 month.
3. The fumarate in crystal form IV was left to stand under 25 °C, 60% RH conditions and 40 °C, 75% RH conditions to investigate its stability.

**Table 31**

| | Conditions of sitting | Purity (%) | Purity (%) | Crystal form |
|---|---|---|---|---|
| | | Initial | 1 month | |
| Sample | 25°C, 60%RH | 99.46 | 99.40 | IV |
| | 40°C, 75%RH | | 99.34 | IV |

The long-term/accelerated stability study shows that the fumarate in crystal form IV has good physical and chemical stability when left to stand under long-term and accelerated stability conditions for 1 month.
4. The fumarate in crystal form II was subjected to 6-month long-term (25 °C, 60% RH) and accelerated (40 °C, 75% RH) stability studies. The results are shown in the table below.

**Table 32. The results of the long-term and accelerated stability studies for the fumarate in crystal form II**

| Sample | Conditions of sitting | Initial | Purity (%) 1 month | Purity (%) 2 months | Purity (%) 3 months | Purity (%) 6 months | Crystal form |
|---|---|---|---|---|---|---|---|
| Crystal form II | 25°C, 60%RH | 99.7 | 99.7 | 99.7 | 99.7 | 99.7 | II |
| | 40°C, 75%RH | 99.7 | 99.7 | 99.7 | 99.7 | 99.7 | II |

Conclusion: the results show that the crystal form II sample of the fumarate of the compound of formula 1 has good physical and chemical stability when left to stand under long-term (25 °C, 60% RH) and accelerated (40 °C, 75% RH) conditions for 6 months.

### Example 25: Preparation of Fumarate in Amorphous Form

500 mg of the free base of the compound of formula (I) was dissolved in 10 mL of methanol, and 1 eq of fumaric acid and 60 acid were added. The mixture was stirred for 30 min and concentrated to dryness by rotary evaporation to give a solid. The solid was identified as amorphous by XRPD.

## Claims

1. A pharmaceutically acceptable salt of a compound of formula (I), wherein the pharmaceutically acceptable salt is selected from the group consisting of a fumarate, an oxalate, a succinate, a maleate, a hydrochloride, a malate, and a hydrobromide,

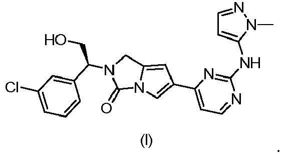

2. A fumarate of the compound of formula (I) according to claim 1, wherein the fumarate of the compound of formula (I) comprises the compound of formula (I) and fumaric acid in a molar ratio of 1:1 or 2:1.

3. A crystal form I of a fumarate of a compound of formula (I), wherein 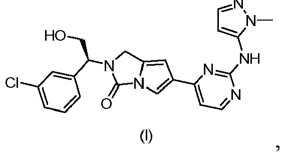 the fumarate of the compound of formula (I) comprises the compound of formula (I) and fumaric acid in a molar ratio of 1:1 and, in an X-ray powder diffraction graph, has characteristic peaks at 2θ angles of diffraction of 10.671, 22.735, 28.681, and 29.302.

4. A crystal form II of a fumarate of a compound of formula (I), wherein 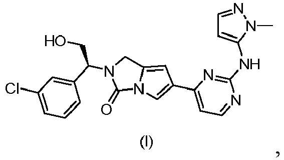 the fumarate of the compound of formula (I) comprises the compound of formula (I) and fumaric acid in an amount-of-substance ratio of 1:1 and, in an X-ray powder diffraction graph, has characteristic peaks at 2θ angles of diffraction of 7.783, 9.151, 12.378, 14.808, 18.574, 25.755, and 26.802.

5. A crystal form III of a fumarate of a compound of formula (I), wherein 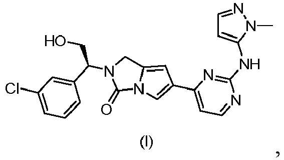 the fumarate of the compound of formula (I) comprises the compound of formula (I) and fumaric acid in a molar ratio of 2:1 and, in an X-ray powder diffraction graph, has characteristic peaks at 2θ angles of diffraction of 4.877, 10.601, 13.827, 14.867, 16.272, 18.874, 23.921, and 26.367.

6. A crystal form IV of a fumarate of the compound of formula (I) according to claim 1, wherein the fumarate of the compound of formula (I) comprises the compound of formula (I) and fumaric acid in a molar ratio of 2:1 and, in an X-ray powder diffraction graph, has characteristic peaks at 2θ angles of diffraction of 4.854, 10.613, 13.692, 23.523, 24.252, and 25.925.

7. A hydrobromide of the compound of formula (I) according to claim 1, wherein the compound of formula (I) and hydrobromic acid are in a molar ratio of 1:1 or 1:2.

8. A crystal form α of a hydrobromide of a compound of formula (I), wherein 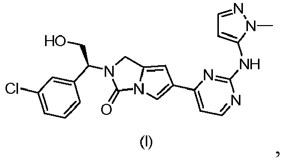 the hydrobromide of the compound of formula (I) comprises the compound of formula (I) and hydrobromic acid in a molar ratio of 1:1 and, in an X-ray powder diffraction graph, has characteristic peaks at 2θ angles of diffraction of 6.656, 9.552, 14.110, 19.095, 20.092, 25.220, and 27.119.

9. A crystal form β of a hydrobromide of a compound of formula (I), wherein 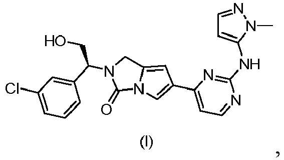 the hydrobromide of the compound of formula (I) comprises the compound of formula (I) and hydrobromic acid in a molar ratio of 1:2 and, in an X-ray powder diffraction graph, has characteristic peaks at 2θ angles of diffraction of 7.363, 12.914, 15.392, 16.865, 17.217, 19.704, 21.999, 22.515, 24.769, 27.056, and 28.236.

10. The crystal form of the fumarate and the crystal form of the hydrobromide of the compound of formula (I) according to any one of claims 3-6 and 8-9, wherein the 2θ angles have a margin of error of ±0.3, preferably ±0.2.

11. A pharmaceutical composition, comprising the following components:
i) the pharmaceutically acceptable salt of the compound of formula (I) or the crystal form thereof according to any one of claims 1-10, or a mixture thereof, and
ii) one or more pharmaceutically acceptable carriers, diluents, or excipients.

12. A method for preparing a composition comprising a pharmaceutically acceptable salt of a compound of formula (I) or a crystal form thereof, or a mixture thereof, comprising a step of mixing the pharmaceutically acceptable salt of the compound of formula (I) or the crystal form thereof, or the mixture thereof, with one or more pharmaceutically acceptable carriers, diluents, or excipients.

13. Use of the pharmaceutically acceptable salt of the compound of formula (I) or the crystal form thereof according to any one of claims 1-10, or a mixture thereof, or the pharmaceutical composition according to claim 11 in preparing a medicament for treating or preventing cancer, inflammation, or other proliferative diseases, preferably cancer, wherein the cancer is selected from the group consisting of melanoma, liver cancer, kidney cancer, lung cancer, nasopharyngeal carcinoma, colorectal cancer, colon cancer, rectal cancer, pancreatic cancer, cervical cancer, ovarian cancer, breast cancer, bladder cancer, prostate cancer, leukemia, head and neck squamous cell carcinoma, cervical cancer, thyroid cancer, lymphoma, sarcoma, neuroblastoma, brain tumor, myeloma, astrocytoma, and glioma.

14. A method for preparing fumaric acid of a compound of formula (I), comprising a step of reacting the compound of formula (I) with fumaric acid.
